# EUROPEAN PATENT APPLICATION

(11) **EP 4 104 830 A1**
(43) Date of publication of application: **21.12.2022**
(21) Application number: 21179848.3
(22) Date of filing: 16.06.2021
(51) Int. Cl.: A61K 31/282, A61K 31/352, A61K 31/475, A61K 31/713, A61K 33/243, A61K 39/395, A61P 35/00

(54) **SEQUENTIAL INNATE AND ADAPTIVE IMMUNE MODULATION FOR CANCER TREATMENT**

(71) Applicant: Wittig, Burghardt, 14129 Berlin (DE)
(72) Inventor: Wittig, Burghardt, 14129 Berlin (DE)
(74) Representative: Schulz Junghans Patentanwälte PartGmbB

(57) **Abstract**

The invention relates to the use of TLR9 agonists, and checkpoint modulators in biomarkerled treatment protocols designed to deliver optimal immune modulation signals to cancer patients following an induction chemotherapy treatment with a chemotherapeutic dug.

## Description

The present invention relates to a TLR9 agonist, and a checkpoint modulator agent for use in treating cancer patients.

### Background of the Invention

Toll-like receptors (TLR) belong to the group of pattern recognition receptors recognising pathogen-associated molecular patterns like lipopolysaccharides, or pathogen-specific nucleic acids, which are ubiquitous in pathogens. TLR play a key role in immediate immune responses by enabling immune cells to fight pathogens via the innate immune system. Adaptive immunity in turn activates the antigen-specific effector T cells, and memory T cells of the adaptive immune arm. Like checkpoint inhibitors, TLR agonists are thus attractive candidates for the development of therapeutic immune modulators to treat cancer.

TLR9 is predominantly expressed by plasmacytoid dendritic cells (pDC) and B cells. It recognizes non-methylated CG- motifs absent in human/vertebrate nuclear DNA, and broadly activates both innate and adaptive immunity. Synthetic DNA-molecules are being used for immunotherapeutic approaches, containing non-methylated CG-motifs which function as TLR9 agonists by mimicking the DNA of pathogens to trigger a wide range of immunological activity.

Members of the dSLIM^{®} family (dSLIM: double Stem Loop Immunomodulator), a family of TLR9 agonists, consist of dumbbell-shaped, covalently closed DNA molecules devoid of any chemical or other artificial modifications of the DNA (Kapp K. et al. 2019 Oncoimmunology DOI: 10.1080/2162402X.2019.1659096, Schmidt M. et al 2015 Nucleic Acid Therapeut. 25(3):140). Protection against nucleolytic degradation is achieved by the covalently closed structure avoiding accessible 3'- or 5'-ends. Lefitolimod (disclosed in US6849725B2, incorporated by reference herein in its entirety; elsewhere referred to as MGN1703) belongs to this group of TLR9 agonists exhibiting a specific immunomodulatory sequence and structure. Promising immunogenicity data in preliminary murine studies and exploratory studies in combination with chemotherapy in metastatic colorectal carcinoma (mCRC) and extensive stage small cell lung cancer (esSCLC) suggests TLR9-triggered immune activation can be harnessed to reactivate immune surveillance to recognize tumour-associated, tumour-specific, and tumour-neo antigens on tumour cells of cancer patients, however it is not yet clear which subsets of cancer patients, or which clinical treatment context is associated with enhanced overall survival (OS) in response to TLR9 agonist treatment.

Based on the above-mentioned state of the art, the objective of the present invention is to provide dosing schedules and combinations comprising TLR9 agonist and checkpoint modulation compounds capable of modulating the innate or adaptive immune response, in order to treat cancer patients. This objective is attained by the subject-matter of the independent claims of the present specification, with further advantageous embodiments described in the dependent claims, examples, figures and general description of this specification.

### Summary of the Invention

The invention provides TLR9 agonist, or checkpoint modulator (CPM) drugs, for use in subgroups of cancer patients. Experimental studies herein demonstrate the presence of immune correlates in blood samples, indicating whether a patient is likely to be responsive to innate or adaptive immune stimulation, following a first induction chemotherapy treatment. In addition, the invention provides specific timing, and treatment conditions, for administration of said formulations which the inventors find to be associated with improved clinical outcomes.

One aspect of the invention provides a TLR9 agonist for use in patients, having first received an induction chemotherapy treatment for cancer, and having been found to display markers of immune cell subset activation below a threshold median value indicating immune exhaustion, in a first predictive biomarker assay. Subsequently, the TLR9 agonist is first administered in the form of 1 cycle, or 2, or 3 repetitions of a *TLR9 agonist induction immunomodulation cycle* (wherein a chemotherapy agent is administered the first week, and the TLR9 agonist is administered in a second, and a third week). It is then administered as part of a *TLR9 agonist maintenance immunomodulation regimen* (where the TLR9 agonist is administered twice per week, once per each week, or every two weeks for n weeks of said TLR9 agonist maintenance immunomodulation regimen, wherein n is an integer from 5 to 35 weeks, particularly from 9 to 18 weeks, more particularly where n is 12 weeks).

Another aspect of the invention provides a CPM drug for use in patients having received an induction chemotherapy treatment for cancer, and displaying markers of immune cell subset activation above a threshold median value, indicating immune exhaustion in a first predictive biomarker assay. Following this, the CPM agent is first administered in the form of a 1 to 5 sets of a *CPM agent induction immunomodulation cycle* (wherein a chemotherapy agent and the CPM drug are administered together once every three weeks). It is subsequently administered as part of a *CPM agent maintenance immunomodulation regimen* (where the CPM agent is administered twice per week, once per each week, or every two weeks of said CPM maintenance immunomodulation regimen for n weeks, wherein n is an integer from 6 to 36 weeks, particularly from 9 to 18 weeks, more particularly n is 12 weeks).

One aspect of the invention relates to the assignment of cancer patients to alternations of immunomodulation treatment following induction chemotherapy, with either a TLR9 agonist, or a CPM drug. The timing of said alternating treatment is led by the outcome of a first, and a second predictive biomarker assay measuring indicators of immune exhaustion.

In another embodiment, the present invention relates to a pharmaceutical composition comprising at least one of the compounds of the present invention, or a pharmaceutically acceptable salt thereof, and at least one pharmaceutically acceptable carrier, diluent or excipient.

### Terms and definitions

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising," "having," "containing," and "including," and other similar forms, and grammatical equivalents thereof, as used herein, are intended to be equivalent in meaning and to be open ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictate otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a," "or," and "the" include plural referents unless the context clearly dictates otherwise.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic and biochemical methods (see generally, Sambrook et al., Molecular Cloning: A Laboratory Manual, 4th ed. (2012) Cold Spring Harbour Laboratory Press, Cold Spring Harbour, N.Y. and Ausubel et al., Short Protocols in Molecular Biology (2002) 5th Ed, John Wiley & Sons, Inc.) and chemical methods.

### Predictive Biomarker assays

Predictive biomarkers according to the invention assay the cell surface expression of immune cell subset phenotyping markers, and activation markers in peripheral blood mononuclear cells (PBMC) isolated from blood samples, in order to determine the immune status of a cancer patient. The expression of an activation marker, or a phenotyping marker may be assayed via techniques such as flow cytometry, or multiplex analyses.

Appropriate methods to measure activated immune cell populations in liquid biopsies are known in the art, including methodologies which determine cell phenotype, and activation marker expression at a single cell level, on slides or in liquid samples, to obtain the proportion of activated cells within a specified cell subset. Such methodologies include multiplexed immunohistochemistry, flow cytometry protocols, or mass spectrometry assays carried out by either histology, or fluidics-based assays. Flow cytometry is a method of particular use to measure cell subsets, and their activation markers according to the invention.

In the present specification, the term *positive,* when used in the context of expression of a marker, refers to expression of an antigen assayed by a labelled molecular probe, particularly a fluorescently labelled antibody, wherein the label's signal (e.g. fluorescence) on the structure (for example, a cell) referred to as "positive" is at least 30% higher (≥ 30 %), particularly ≥50% or ≥80%, in median intensity in comparison to staining with an isotype-matched labelled antibody which does not specifically bind to the same target. Such expression of a marker is indicated by a superscript "plus" (⁺), following the name of the marker, e.g. CD4⁺, or by the addition of the plus sign after the marker name ("CD4+"). If the word "expression" is used herein in the context of "gene expression" or "expression of a marker or biomolecule" and no further qualification of "expression" is mentioned, this implies "positive expression" as defined above.

In the present specification, the term *negative,* when used in the context of expression of a marker, refers to expression of an antigen assayed by a labelled molecular probe, particularly a fluorescently labelled antibody, wherein the median label signal intensity is less than 30% higher, particularly less than 15% higher, than the median intensity of an isotype-matched antibody which does not specifically bind the same target. Such expression of a marker is indicated by a superscript minus (⁻), following the name of the marker, e.g. CD86⁻, or by the addition of the minus sign after the marker name ("CD86-").

The term *molecular probe* in the context of the present specification relates to a specific ligand, particularly an antibody, antibody fragment, an antibody-like molecule or aptamer, more particularly an antibody or antibody fragment, that can bind to a target molecule, such as a specific surface protein, with a dissociation constant of ≤10⁻⁷ mol/l, particularly ≤10⁻⁸ mol/l. The molecular probe comprises a detectable marker such as a fluorescent dye, metal particle, bead, dye or enzyme.

The term *set of molecular probes* relates to a panel of molecular probes specific for the cell phenotype markers or activation markers measured in the predictive biomarker assay.

The term *fluorescent label, or fluorescent dye* in the context of the present specification relates to a small molecule capable of fluorescence in the visible or near infrared spectrum. Examples for fluorescent labels or labels presenting a visible colour include, without being restricted to, fluorescein isothiocyanate (FITC), rhodamine, allophycocyanin (APC), peridinin chlorophyll (PerCP), phycoerythrin (PE), Alexa Flours (Life Technologies, Carlsbad, CA, USA), dylight fluors (Thermo Fisher Scientific, Waltham, MA, USA) ATTO Dyes (ATTO-TEC GmbH, Siegen, Germany), BODIPY Dyes (4,4-difluoro-4-bora-3a,4a-diaza-s-indacene based dyes) and the like.

### Chemotherapy treatment

The term *induction chemotherapy* in the context of the present specification relates to a period of weeks or months, particularly 6 weeks to 12 weeks, where a cancer patient is administered a standard of care chemotherapy drug, i.e. an antineoplastic chemotherapy drug, in order to induce tumour cell death. This releases tumour antigens which initiate an innate and adaptive immune response to cancer cells, which can later be harnessed by administration of the immunomodulatory agents provided by the invention, namely TLR9 agonist molecules, or check modulator agents (CPM). Such *induction chemotherapy* typically comprises, or consists of, repeated "cycles" of treatment, each cycle consisting of administration of an antineoplastic chemical formulation, or a targeted monoclonal antibody treatment, once in a period of 2 to 4 weeks, usually once every 3 weeks. The antineoplastic agent used in the *induction chemotherapy* is not particularly limited according to the invention and should be chosen, and prescribed according to the type and grade of tumour, as ascertained by standard of care clinical assessments.

Examples of induction chemotherapy include, but are not limited to, in the case of esSCLC patients such as those participating in the IMPULSE trial, 2 to 6 cycles of platinum drug-based combination induction chemotherapy, each cycle comprising administration of cisplatin or carboplatin, in combination with etoposide, once every three weeks. In the case of breast cancer, or cervical cancer, induction chemotherapy may consist of cycled administrations of a platinum complex such as cisplatin, or carboplatin, or an epidermal growth factor or vascular growth factor-inhibiting antibody.

The term *induction chemotherapy* as used herein encompasses so called "re-induction" chemotherapy studied in the IMPALA trial according to mCRC treatment guidelines, wherein said chemotherapy drug treatment protocol is being provided to a patient for a second time after a period of remission, in the case of tumour recurrence, and/or the diagnosis of metastasis. In the case of metastatic colorectal cancer patients, such as those participating in the IMPALA clinical trial, an induction (or reinduction) chemotherapy may be cycles of treatment with an antineoplastic agent such as, but not limited to, an antibody targeting growth hormone receptor, platinum-based chemotherapy, platinum complexes such as oxaliplatin, or other anti-mitotic chemotherapeutic agents.

The term *platinum-based chemotherapy treatment* as used in the current specification, refers to a combination medicament comprising
- a platinum-containing drug, also known as a platinum-containing complex, for example a platinum-containing drug selected from carboplatin, satraplatin, cisplatin, dicycloplatin, nedaplatin, oxaliplatin, picoplatin, and/or triplatin,
in combination with a second chemotherapeutic agent.

The platinum drug and the second chemotherapeutic agent may be either combined in a single tablet, or infusion, or delivered simultaneously as two separate preparations.

Examples of additional antineoplastic chemotherapy drugs which may be utilised in *induction chemotherapy* cycles, or as second chemotherapeutic drugs which may be used together with a platinum-containing drug as specified above to provide a platinum-based chemotherapy treatment, are known in the art, and include, but are not limited to the following drug classes:
- an alkylating antineoplastic drug, particularly an alkylating antineoplastic drug selected from altretamine, bendamustine, busulfan, carmustine, cyclophosphamide, chlorambucil, dacarbayine, etoposide, ifosfamide, lomustine, mechlorethamine, melphalan, oxaliplatin, temozolomide, thiptepa, and trabectedin;
- an antimetabolite-type antineoplastic drug, particularly an antimetabolite-type antineoplastic drug selected from; azacytidine, 5-fluorouracil, 6-mercaptopurine, capecitabine, clofarabine, cytarabine, decitabine, floxuridine, fludarabine, gemcitabine, hydroxyurea, hydroxycarbamide, methotrexate, nelarabine, pemetrexed, pentostatin, pralatrexate, and phototrexate;
- a mitotic inhibitor-type antineoplastic drug, particularly a mitotic inhibitor-type antineoplastic drug selected from capazitaxel, docetaxel, nab-paclitaxel, paclitaxel, vinblastine, vincristine, and vinorelbine;
- an antimetabolite-type antineoplastic drug, particularly an antimetabolite-type antineoplastic drug selected from; azacytidine, 5-fluorouracil, 6-mercaptopurine, capecitabine, clofarabine, cytarabine, decitabine, floxuridine, fludarabine, gemcitabine, hydroxyurea, hydroxycarbamide, methotrexate, nelarabine, pemetrexed, pentostatin, pralatrexate, and phototrexate;
- an anthracycline-type antineoplastic drug, particularly an anthracycline-type antineoplastic drug selected from daunorubicin, doxorubicin, epirubicin, and idarubicin;
- a selective SERM antineoplastic drug, particularly a SERM drug selected from raloxifene, toremifene and tamoxifen;
- a SERD antineoplastic drug, particularly a SERD antineoplastic drug selected from fulvestrant, brilandestrant and elacestrant;
- an aromatase inhibitor drug, particularly an aromatase inhibitor antineoplastic drug selected from exemestane, letrozole, vorozole, formestane, fadrozole and anastrozole; and/or
- a PI3K pathway inhibitor drug, particularly a PI3K pathway inhibitor drug selected from rapamycin, dactolisib, BGT226, SF1126, PKI-587, and NVPBE235;
- a HER2 targeting antineoplastic drug, particularly a HER2 targeting antineoplastic drug selected from trastuzumab, pertuzumab, SYD985, RC48, A166, HER2ALT-P7, T-DM1, ARX788, KN026, BVAC-B, MT-5111, AVX901, TAS0728, MP0274, MM-302, FS102, and H2NVAC;
- an inhibitor of oestrogen growth factor receptor (EGFR) bioactivity antineoplastic drug, particularly an inhibitor of EGFR bioactivity antineoplastic drug selected from gefetinib, erlotinib, lapatinib, cetuxumib, neratinib, osimeratib, panitumamib, vandetanib, necitumumab, and dacomitinib;
- an antiangiogenic antineoplastic drug, particularly an antiangiogenic antineoplastic drug selected from bevacizumab, itraconazole, thalidomide, and lenalidomide, or an inhibitor of vascular endothelial growth factor (VEGF) bioactivity antineoplastic drug.

The term *sets of cycles, treatment cycles,* or cycles in the context of the current specification refers to those treatments where more than one drug, or combinations or drugs are delivered in a specific order over a specified time period, and this order is optionally repeated in identical sets or cycles or treatment. The term *regimen* as used herein refers to a single treatment, or therapeutic agent, delivered at regular intervals for a specified period of weeks.

### Immunotherapy

In the context of the present specification, the terms *cancer immunomodulation, cancer immunotherapy, biological* or *immunomodulatory therapy* are meant to encompass types of cancer treatment that help the immune system to fight cancer. Non-limiting examples of cancer immunotherapy include immune checkpoint inhibitors and agonists, T cell transfer therapy, cytokines and their recombinant derivatives, adjuvants, and vaccination with small molecules or cells.

In the context of the present specification, the term *checkpoint modulator* or *checkpoint inhibitor antibody* is meant to encompass a cancer immunotherapy agent, particularly an antibody (or antibody-like molecule), capable of disrupting an inhibitory signalling cascade that limits immune cell activation, known in the art as an immune checkpoint mechanism. In the context of the present specification, the term *checkpoint modulator* is meant to encompass an agent, particularly an antibody (or antibody-like molecule) capable of disrupting the signal cascade leading to T cell inhibition after T cell activation as part of what is known in the art the immune checkpoint mechanism. In certain embodiments, the *checkpoint inhibitory agent* or *checkpoint inhibitor antibody* is an antibody to CTLA-4 (Uniprot P16410), PD-1 (Uniprot Q15116), PD-L1 (Uniprot Q9NZQ7), B7H3 (CD276; Uniprot Q5ZPR3), VISTA (Uniprot Q9H7M9), TIGIT (UniprotQ495A1), TIM-3 (HAVCR2, Uniprot Q8TDQ0), CD158 (killer cell immunoglobulin-like receptor family), TGF-beta (P01137).

Non-limiting examples of a *checkpoint modulator agents* or *checkpoint inhibitory antibodies* include antibodies to CTLA-4 (Uniprot P16410) such as exemplified by ipilimumab (Yervoy; CAS No. 477202-00-9), or antibodies to PD-1 (Uniprot Q15116) or to PD-L1 (Uniprot Q9NZQ7), B7H3 (CD276; Uniprot Q5ZPR3), exemplified by the clinically available antibody drugs nivolumab (Bristol-Myers Squibb; CAS No 946414-94-4), Durvalumab (Astra Zeneca, CAS No. 1428935-60-7), pembrolizumab (Merck Inc.; CAS No. 1374853-91-4), pidilizumab (CAS No. 1036730-42-3), atezolizumab (Roche AG; CAS No. 1380723-44-3), Cemiplimab (Sanofi Aventis; CAS No. 1801342-60-8) and Avelumab (Merck KGaA; CAS No. 1537032-82-8).

In the context of the present specification, the term *TLR9 agonist* refers to a stimulant of the toll-like receptor 9 which detects pathogenic DNA, particularly members of the dSLIM^{®} family (dSLIM: double Stem Loop Immunomodulator), a new family of TLR9 agonists, consisting of dumbbell-shaped, covalently closed DNA molecules devoid of any chemical or other artificial modifications of the DNA (Kapp K. et al. 2019 Oncoimmunology DOI: 10.1080/2162402X.2019.1659096, Schmidt M. et al 2015 Nucleic Acid Therapeut. 25(3):140). Protection against nucleolytic degradation is achieved by the covalently closed structure avoiding accessible 3' ends. Lefitolimod (MGN1703) belongs to this group of TLR9 agonists exhibiting a specific immunomodulatory sequence and structure.

As used herein, the term *pharmaceutical composition* refers to a compound of the invention, or a pharmaceutically acceptable salt thereof, together with at least one pharmaceutically acceptable carrier. In certain embodiments, the pharmaceutical composition according to the invention is provided in a form suitable for topical, parenteral or injectable administration.

As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavouring agents, dyes, and the like and combinations thereof, as would be known to those skilled in the art (see, for example, Remington: the Science and Practice of Pharmacy, ISBN 0857110624).

As used herein, the term *treating* or *treatment* of any disease or disorder (e.g. cancer) refers in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

### Detailed Description of the Invention

### TLR9 agonists in sequential application / alternation schedules

A first aspect of the invention relates to a TLR9 agonist for use in treating a patient diagnosed with cancer.

The TLR9 agonist is administered to a patient who has received an induction chemotherapy, consisting of a first set of chemotherapy drug treatment cycles. Induction chemotherapy refers to a period of weeks to several months, particularly 6 to 12 weeks of standard of care antineoplastic drug treatment suited to the patient tumour type, usually administered in cycles where the drug is administered orally, or by infusion every 2 to 4 weeks. This induction chemotherapy encompasses both primary cancer diagnosis treatment, and re-induction chemotherapy administered to patients who have already received cancer treatment in the past, and have recently been diagnosed with a recurrence of cancer, or cancer progression after a period of disease , or lack of tumour growth above a baseline measure, or threshold.

Subsequent to the induction chemotherapy (subsequent to in the context of the invention means preferably in the week subsequent to induction chemotherapy, or at most beginning two or three weeks afterwards), the TLR9 agonist is administered as part of a *TLR9 agonist induction immunomodulation cycle.*

According to this aspect of the invention, the *TLR9 agonist induction immunomodulation cycle* comprises or consists of administration of a chemotherapeutic agent or drug, or a drug combination, in a first week of said TLR9 agonist induction immunomodulation cycle, together with a dose of a TLR9 agonist. This is followed by administration of the TLR9 agonist once per week, or twice per week in a second, and a third week of said TLR9 agonist induction immunomodulation cycle. In some embodiments, the TLR9 agonist induction immunomodulation cycle utilizes the same treatment agent administered in the induction chemotherapy phase. The chemotherapeutic agent may be selected from those listed used in induction chemotherapy above. Particular embodiments relate to the use of a platinum-containing complex, or a platinum-based chemotherapy treatment, in combination with the TLR9 agonist.

Subsequent to 1, 2, 3, or 4 repeated cycles of said TLR9 agonist induction immunomodulation cycles, the TLR9 agonist is sequentially administered as part of a *TLR9 agonist maintenance immunomodulation regimen.*

According to this aspect of the invention, during the *TLR9 agonist maintenance immunomodulation regimen* the TLR9 agonist is administered twice per week, once per each week, or every two weeks for n weeks of said TLR9 agonist maintenance immunomodulation regimen, wherein n is an integer from 5 to 35 (encompassing treatment schedules suitable to mCRC, mBC or to SCLC and other cancers). In particular embodiments, n is is an integer from 9 to 18 weeks (standard treatment timing relating to SCLC). In more particular embodiments, n is 12 weeks (demonstrated to comprise a population of responsive patients in the IMPULSE trial assessed in the examples). In contrast to the *TLR9 agonist induction immunomodulation* cycle, the patient is not administered additional chemotherapeutic agents during the *TLR9 agonist maintenance immunomodulation regimen.*

Particular embodiments of this aspect of the invention relate to a TLR9 agonist for use in a patient who has been assigned a status of "non-matched-in-first" in a first predictive biomarker assay. The first predictive biomarker assay determines an immunity status in a blood sample obtained from the patient which measures immune exhaustion, namely a percent of activated cells within an immune cell subset selected from B cells, T cells, natural killer T (NKT) cells, dendritic cells (DC), myeloid dendritic cells (DC), and/or plasmacytoid dendritic cells (pDC), or the percent of T regulatory (Treg) cells in the CD4+ T cell subset. In particular embodiments, the predictive biomarkers assay according to the invention utilises flow cytometry to identify cell subsets, and measure the proportion which display an activated phenotype.

The percent of activated immune cells, or Treg cells determined in the first predictive biomarker is compared to a threshold of a median value previously determined for blood samples obtained from a cohort of cancer patients having been diagnosed with the same cancer as the patient, and having undergone said first set of treatment cycles prior to biomarker assessment. A patient is assigned a "matched" status if the proportion of activated immune cells is at, or above this median threshold, and a "non-matched" status if the percent of activated cells, or Treg cells, is below the threshold.

Certain embodiments of the first predictive biomarker relate to the determination of the percent of T regulatory (Treg) cells among CD4+ T cells. According to these embodiments, the patient is assigned a status of "non-matched-in-first" if the percent of activated cells within the immune subset is below (<) a median percent of activated cells, or < a median percent of Treg cells among CD4+ T cells.

Particular embodiments of the first predictive biomarker according to the first aspect of the invention relate to the use of a flow cytometry assay which determines the percent of B cells (for example defined as CD19+ CD45+ cells) which express an activation marker such as CD80, or CD86, particularly CD86 (Uniprot P42081). In some embodiments the patient is assigned a status of "non-matched-in-first" if the percent of activated B cells is below a threshold of about 16.9%.

High expression of immune activation markers assayed in the first, or the second predictive biomarker assay suggests a patient has received sufficient innate immune stimulant TLR9 agonist, are thus more likely to benefit from an alternative treatment, for example a chemotherapeutic drug, or particularly a checkpoint modulator.

In some embodiments the treatment of a patient having undergone induction chemotherapy and having been assigned a status of "matched-in-first" in a first predictive biomarker assay as specified according to the aspect of the invention related above, is not particularly limited by the invention. A patient may be assigned a status of "matched-in-first" if the percent of activated cells within the immune subset is ≥ said threshold, particularly the median percent of activated cells, or if the percent of Treg cells among CD4+ T cells ≥ a threshold. In particular embodiments, a patient is assigned matched-in-first" above, or equal to a median percent of 16.9% B cells.

In certain embodiments, if a first predictive biomarker is matched, for example, if the percent of activated B cells observed in the test sample is equal to, or exceeds a given threshold of immune activation indicating immune exhaustion, the TLR9 agonist is discontinued. Certain embodiments relate to a return to the standard of care cancer treatment on matching this biomarker, for example continuation maintenance by platinum-based chemotherapy, or switch to a second line chemotherapy such as topotecan as demonstrated in the IMPULSE trial.

**In** certain embodiments, the TLR9 administered to a cancer patient who has received the TLR9 agonist maintenance immunomodulation regimen as specified above in the previous 3 weeks, and who has also undergone a second predictive biomarker assay. In certain particular embodiments, this second predictive biomarker assay determines a percent of T regulatory cells among CD4+ T cells in a blood sample obtained from the patient.

In some embodiments, the patient is assigned a status of "non-matched-in-second" if the percent of activated Treg cells in the sample is < a median Treg obtained from a cohort of patients who have undergone the same treatment for the same condition. In some embodiments, the patient is assigned a status of "non-matched-in-second" if the percent of activated Treg cells in the sample is < 20%.

Subsequent to being assigned a status of "non-matched-in-second", the patient is administered the TLR9 agonist twice per week, once per week, or every 2 weeks for n weeks, wherein n is an integer from 6 to 24. In particular embodiments, n is an integer from 6 to 15. In more particular embodiments thereof, n is 9.

In certain embodiments, the TLR9 agonist is administered following the induction chemotherapy as above, when in the second predictive biomarker:
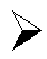 the patient is assigned a status of "non-matched-in-second" if the percent of Treg cells among CD4+ T cells in the sample is < 20%, or
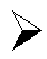 the patient is assigned a status of "matched-in-second" if the percent of Treg cells among CD4+ T cells in the sample is ≥ 20%.

In alternative embodiments,
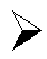 the patient is assigned a status of "non-matched-in-second" if the percent of Treg cells among CD4+ T cells in the sample is < about 15%, or
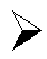 the patient is assigned a status of "matched-in-second" if the percent of Treg cells among CD4+ T cells in the sample is ≥ about 15%.

In alternative embodiments of the second predictive biomarker assay, the patient is assigned a biomarker status on the basis of the percent of activated B cells determined in an *ex vivo* sample, for example, assigned a status of "matched-in-second" if the percent of CD19+ cells which co-express CD80, or CD86, is ≤ threshold of around 15%, and "non-matched-in-second if the percent of activated B cells is < 15 %.

Subsequent to being assigned a status of "non-matched-in-second", the patient continues to be administered a TLR9 agonist twice per week, once per week, or every 2 weeks for n weeks, wherein n is an integer from 6 to 24. In particular embodiments, n ranges from 6 to 15. In more particular embodiments, n is 9. Subsequent to being assigned a status of "matched-in-second", a checkpoint modulator (CPM) is administered once every 2, or 3 weeks for n weeks, wherein n is an integer between 3 to 15.

In some embodiments, the patient is assigned a status of "matched-in-second" if the patient has reached the median progression free survival of a cohort of patients receiving the induction, or re-induction chemotherapy in a clinical standard of care setting.

In the context of the present specification, the terms *tumour progression,* or *disease progression,* refer to tumour growth, relapse, or metastasis in comparison to an index assessment of tumour size, grade, and severity taken at a baseline measurement before induction chemotherapy. Disease progression according to the invention may be assessed using standardised clinical evaluation protocols known in the art, for example the RECIST 1.1 criteria (Lawrence S. H. et al. 2016 Eur. J. Cancer 62:138) or the irRC criteria (Hoos A. et al. 2007 **30**(1)1). Disease progression may be assigned, or diagnosed, for example, upon the identification of new cancer cell lesions, and/or a 25% increase in tumour volume as determined by bidirectional measurements at a timepoint after a baseline measurement.

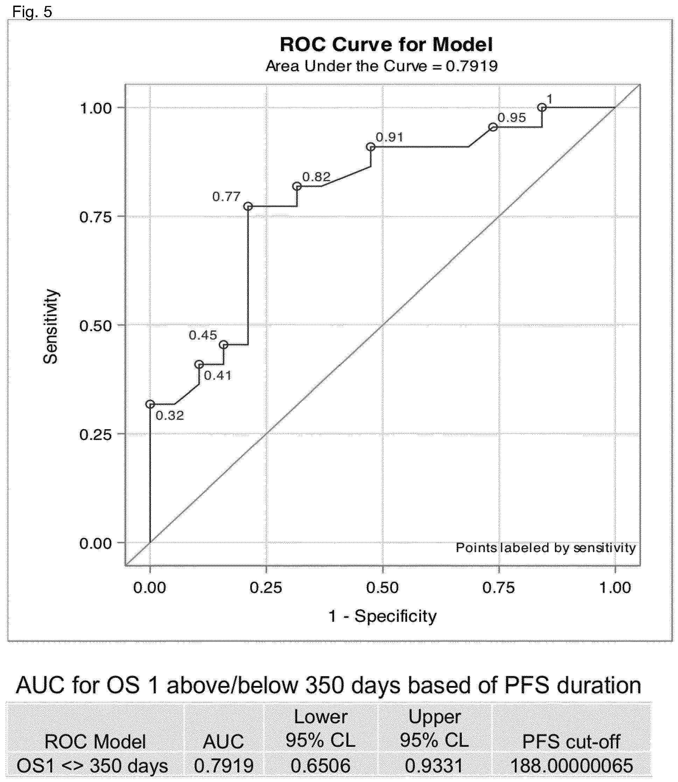

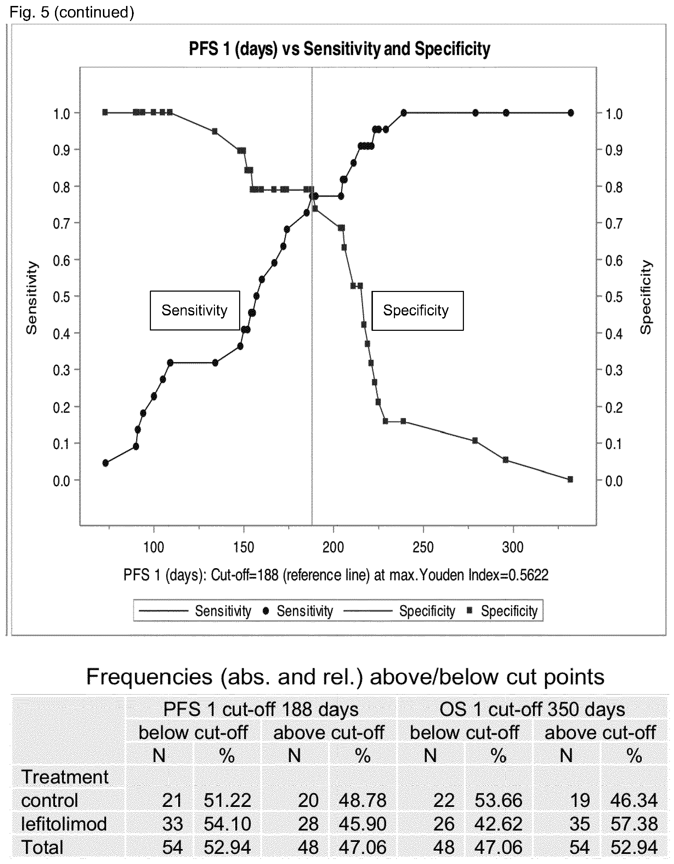

A patient who has not been characterised as having *disease progression* in contrast, is said to be characterised by *progression-free survival (PFS).* This term refers to the period of time, for example, days from the onset of cancer treatment, in which no *disease progression* according to the thresholds defined by the irRC and/or RECIST 1.1 criteria. In other words, the period of time counting from the first dose of the induction chemotherapy drug that initiates patient treatment protocols according to the invention, in which the patient's tumour does no grow or spread above a baseline level, or a threshold from baseline tumour size, for example, no more than a 25% increase in tumour volume. The *median progression free survival* refers to the median number of days before the diagnosis of disease progression is observed/diagnosed in a cohort of patients characterised by a diagnosis with a cancer derived from the same tissue of origin as the cancer patient being treated according to the invention. The size of the cohort should be sufficient to power a calculation of the median and confidence interval which can differentiate between patient outcomes as demonstrated in the examples (Fig. 5).

In some embodiments, an esSCLC patient having received TLR9 agonist maintenance modulation is assigned a status of "matched-in-second" if they are characterised not having being diagnosed with disease progression, more than 188 days since the onset of induction chemotherapy treatment.

In some embodiments, a colorectal cancer patient having received TLR9 agonist maintenance modulation is assigned a status of "matched-in-second", having not been diagnosed with disease progression for the 225 to 250 days since the onset of a first dose of a re-induction chemotherapy.

In some embodiments, the patient having received TLR9 agonist maintenance modulation is assigned a status of "matched-in-second" if the patient is characterized as being free of disease progression at the median progression free survival timepoint for their specific cancer, for example, in the case of SCLC at least 188 days after the onset of the initiating induction chemotherapy, or if the second predictive biomarker shows that the percent of Treg cells is above a threshold of 15%, or even 20%, or if the percent of activated B cells if more than 15%.

Certain embodiments of the TLR9 agonist for use according to the first aspect of the invention specified above relate to use of the TLR9 agonist wherein said second predictive biomarker assay (as specified in on pages 12 and 13) is performed every 6 to 15 weeks, in order to determine the appropriate timing for a switch to a CPM agent. In particular embodiments, the second predictive biomarker assay is performed every 9 weeks. In certain embodiments, the second predictive biomarker is performed every 9 weeks until patient remission or progression. According to these embodiments,
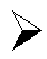 subsequent to being assigned a status of "non-matched-in-second", the TLR9 agonist continues to be administered twice per week, once per week, or every 2 weeks for the next for n weeks, wherein n is an integer in the range from 6 to 24. In particular embodiments n is in the range from 6 to 15. In more particular embodiments, n is 9,
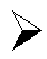 or subsequent to being assigned the status of "matched-in-second", the patient is assigned to switch treatment to a checkpoint modulator (CPM) agent administered once every 2, or 3 weeks for the next 3 to 9 weeks.

In some embodiments, the patient having received a TLR9 agonist subsequent to induction chemotherapy, is assigned to treatment with a checkpoint modulator (CPM) agent administered once every 2, or 3 weeks for the next 3 to 9 weeks, when they have reached a median PFS threshold without a diagnosis of disease progression, without reference to the second predictive biomarker.

In some embodiments, 188 days post induction chemotherapy, an SCLC patient who has been receiving a TLR9 agonist and who has no evidence of disease progression, is switched to treatment with a CPM agent. In alternative embodiments, a mCRC patient receiving TLR9 agonist immunomodulation maintenance regimen, who has reached a threshold of 225 days PFS without a diagnosis of disease progression, is switched to receiving a CPM maintenance immunotherapy regimen.

### TLR9 agonists for use according to the invention

A number of different TLR9 agonist small molecule structures and deoxyribonucleotide oligomers have been developed clinically for use as immunomodulators. Examples include but are not limited to the TLR9 agonist molecules disclosed in US2010144846A1 and family, US2007142315A1 and family, US2004143112A1 and family, US2007066553A1 and family, US2006241076A1 and family, US10894963B2 and family.

In certain embodiments, the TLR9 agonist for use according to the invention comprises at least one unmethylated CG dinucleotide, wherein the CG dinucleotide is part of a sequence N¹N²CGN³N⁴ (a "CpG motif"), wherein N¹N² is AA, TT, GG, GT, or AT, and N³N⁴ is CT, TT, TC, TG, or CG and C is deoxycytidine, G is deoxyguanosine, A is deoxyadenosine, and T is deoxythymidine.

In certain embodiments, the TLR-9 agonist for use according to the invention comprises at least one unmethylated CG dinucleotide, and a stretch of at least three, in particular of four, consecutive deoxyguanosines, wherein the deoxyribose moieties of the oligodeoxyribonucleotide are linked by phosphodiester bonds. In certain particular embodiments thereof, the stretch of at least three, in particular of four, consecutive deoxyguanosines is located at the 5' end of the oligodeoxyribonucleotide.

In certain particular embodiments thereof, a stretch of at least three consecutive deoxyguanosines is located between two CG dinucleotides, more particularly between two CpG motifs.

In certain embodiments, the TLR9 agonist for use according to the invention comprises a double-stranded stem, two single-stranded loops, forms the shape of a dumbbell, and comprises one or several CpG motifs. In particular embodiments thereof, a CpG motif is comprised in the stem part of the dumbbell. In other particular embodiments thereof, a CpG motif is comprised in one or both loop parts of the dumbbell. In yet other particular embodiments thereof, CpG motifs are comprised in both the stem and the loop part of the dumbbell.

In certain embodiments, the TLR9 agonist for use according to the invention is a single circular deoxynucleotide oligomer having a length of 20 to 150, particularly 30 to 60 deoxyribonucleotides.

In certain particular embodiments, the TLR9 agonist for use according to the invention is Lefitolimod (US6849725B2, incorporated by reference herein in its entirety), also referred to as MGN1703 (CAS: 1548439-51-5).

### Checkpoint modulator (CPM) agents in sequential application / alternation schedules

A further aspect of the invention relates to a CPM agent for use in treating cancer, wherein the CPM agent is administered to a patient having first undergone induction chemotherapy consisting of a first set of treatment cycles.

In addition, the patient having undergone induction chemotherapy has been assigned a status of "matched-in-first" in a first predictive biomarker assay measuring immune cell activation in a patient sample as specified on page 10 and 11. The patient is assigned a status of "matched-in-first" if the percent of activated cells within the immune subset is ≥ the threshold values provided for activated immune cells, particularly the median percent of activated cells, or if the percent of Treg cells among CD4+ T cells ≥ a threshold value. In particular embodiments, the CPM is provided for use in a patient assigned a status of "matched-in-first" when the percent of activated B cells determined in a patient sample is above a threshold obtained by determining the median activated B cells in a cohort of patient samples. In particular embodiments, the patient is assigned a "matched-in-first" if the activated B cells in the sample is ≥ a threshold of around 16.9% activated B cells.

Furthermore, subsequent to the induction chemotherapy, the CPM agent is administered as part of a *CPM induction immunomodulation* cycle, particularly a three-week CPM induction immunomodulation cycle. The CPM induction immunomodulation cycle comprises or consists of administration of a chemotherapeutic agent, together with a CPM agent once in the first week of said cycle, followed by no treatment for the subsequent one, or two weeks.

Subsequent to 1 cycle, or 2, 3, 4 or 5 repeated cycles of said CPM induction immunomodulation cycle, the CPM agent is administered as part of a *CPM maintenance immunomodulation regimen,* comprising, or consisting of administration of said CPM agent once every 2 or 3 weeks for n weeks, wherein n is an integer from 6 to 36. In particular embodiments n is an integer from 9 to 18. In more particular embodiments n is 12 weeks administration of the *CPM maintenance immunomodulation regimen.*

In certain embodiments relating to CPM maintenance immunomodulation for treatment of lung cancer, 9 to 18 weeks of said CPM are administered by infusion to a patient having been assigned a status of "matched-in-first" in said first predictive biomarker assay. In particular embodiments, 12 weeks of the CPM maintenance immunomodulation regimen is administered to a lung cancer patient having been assigned "matched-in-first" in the first predicitive assay.

In certain embodiments relating to CPM maintenance immunomodulation administered for treatment of metastatic cancer, particularly mCRC reinduction chemotherapy, the CPM agent is administered for 9 to 18 weeks by infusion in the CPM maintenance immunomodulation regimen. In particular embodiments, said CPM maintenance immunomodulation regimen is administered for 24 weeks.

Certain embodiments of the CPM for use according to this aspect of the invention relate to a patient who has undergone the CPM maintenance immunomodulation regimen for the previous 3 weeks, and where a second predictive biomarker assay value has been determined in a patient sample, where the second predictive biomarker determines the percent of Treg cells among CD4+ T cells in a blood sample obtained from the patient is above a median threshold. In certain embodiments, the patient is assigned a status of "matched-in-second" if the percent of Treg cells among CD4+ T cells is ≥ a threshold from15-20%, and is subsequently administered CPM maintenance immunomodulation regimen where a CPM agent is administered every 2, or 3 weeks for n weeks, wherein n is an integer from 6 to 36. In particular embodiments, n is an integer from 9 to 12. In more particular embodiments, n is 12 weeks administration of the CPM maintenance immunomodulation regimen.

Other embodiments relate to the CPM for use in a patient who has undergone the CPM maintenance immunomodulation regimen as specified in one of the 5 preceding paragraphs, and who has also undergone a second predictive biomarker assay. According to these embodiments:
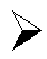 the patient is assigned a status of "non-matched-in-second" if the percent of Treg cells among CD4+ T cells in the sample is < a threshold of 20%,
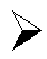 or the patient is assigned a status of "matched-in-second" if the percent of activated Treg cells in the sample is ≥ a threshold of 20%.

In alternative embodiments, the second predictive biomarker threshold is 15% Treg among CD4+ T cells.

Subsequent to being assigned a status of "non-matched-in-second", treatment of the patient who has undergone the CPM maintenance immunomodulation regimen switches to administration of a TLR9 agonist twice per week, once per week, or every 2 weeks for n weeks, wherein n is an integer from 6 to 24. In particular embodiments, n is an integer from 6 to 15. In more particular embodiments n is 9 weeks.

In some embodiments, a patient having received a CPM agent maintenance immunomodulation regimen, subsequently switches to administration of a TLR9 agonist as above, once the patient reaches a threshold of a median PFS survival that characterises their specific cancer disease without application, without reference to a second predictive biomarker assessment. In one particular embodiment, this switch is made after 188 days PFS for an esSCLC patient, or after 225 days PFS for a mCRC patient.

In alternative embodiments, subsequent to being assigned the status of "matched-in-second", the CPM agent continues to be administered to the patient who has undergone the CPM maintenance immunomodulation regimen once every 2, or 3 weeks for n weeks, wherein n is an integer in the range from 6 to 36. In particular embodiments n ranges from 9 to 12 weeks. In more particular embodiments, the patient assigned a status of "matched-in-second" is administered the CPM for 12 weeks.

### Alternating Biomarker-led immunomodulation

Platinum-based induction chemotherapy generates two populations of patient samples derived from tumour patients as defined by the first biomarker. One with immunologically cold tumours, i.e. respective fractions of innate and adaptive immune cell activations ≤ the threshold median biomarker value, the other with immunologically hot tumours, i.e. respective fractions of innate and adaptive immune cell activations > the threshold median biomarker value (though their tumours are immunologically "hot", the underlying immune reactions may no longer be efficacious in terms of anti-tumour activities due to immune exhaustion mediated by checkpoint inhibition pathways).

In the treatment algorithms described according to the invention, immune responses are encouraged in tumours lacking biomarkers of immune cell activation, sometimes referred to as immune "cold" tumours. These are converted in immune "hot" tumours during induction immunomodulation by the subsequent cycles employing chemotherapy in combination with a TLR9 agonist. Once cold tumours are converted into, and maintained as immune hot tumours, the inventors conclude from the evidence demonstrated in the examples that natural counter-regulation through Treg, and B regulatory populations represented by the CD86+ B cell population may be initiated. Therefore, when one or more of the patient sample activation markers match the second biomarker, maintenance immunomodulation is alternated from TLR9 agonist maintenance treatment to CPM treatment according to the treatment regime, with the aim of restarting mechanisms for presentation and cross-presentation of neo-antigens for efficacious anti-tumour adaptive immunity. In repeated application on the second biomarker in intervals of several months, if the patient sample values match the second biomarker once more, maintenance immunomodulation is alternated from the CPM maintenance treatment to TLR9 agonist treatment.

Rather than combining innate and adaptive immunity stimulus according to the prior art, in some embodiments, the TLR9 agonist and CPM agent are delivered in a sequential mode, i.e., through timed, and quantitatively controlled stimulation of the alternative innate and adaptive immune pathways.

In certain embodiments, the second biomarker determines the appropriate timing for switching administration, or repeated, alternating administration of these adaptive (CPM agent) or innate (TLR9 agonist) immune stimulant compounds. According to these embodiments, the CPM, or the TLR9 agonist are administered to a patient who has had said second predictive biomarker assay determined in a blood sample every 6 to 15 weeks, particularly every 9 weeks. If the results of the second predictive biomarker assay indicate that their immune system exhibits signs of exhaustion in the form of activated B cells, or Treg cells above a threshold, the CPM is administered to counter inhibitor checkpoint signals on adaptive immune cells. Alternatively, a TLR9 agonist is administered in an effort to convert a immune "cold" tumour into an active immune environment.

In alternative embodiments, the administration of the adaptive or innate immune stimulants specified in the paragraph above, is alternated when the patient meets the "matched-in-second" criteria, or when the patient reaches the median progression free survival of patients receiving standard of care chemotherapy treat, for example, in the case of SCLC, 188 days of progression free survival, counting from the beginning of the induction chemotherapy cycles, whichever event comes first.

In certain embodiments, where following application of the first predictive biomarker, the patient has undergone the *CPM maintenance immunomodulation regimen* in the previous 3 weeks, then
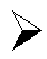 subsequent to being assigned a status of "matched-in-second" (or upon reaching a median threshold for PFS), the TLR9 agonist is administered twice per week, once per week, or every 2 weeks for the subsequent n weeks, wherein n is an integer within the range from 6 to 24. In particular embodiments, n is within the range of 6 to 15 weeks. In more particularl embodiments, n is 9 weeks of TLR9 agonist administration, or
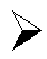 subsequent to being assigned the status of "non-matched-in-second", the CPM continues to be administered once every 2, or 3 weeks for the subsequent n weeks, wherein n is an integer within the range of 6 to 24 weeks. In particular embodiments 9 is within the range of 6 to 12 weeks. In more particular embodiments, n is 9 weeks.

Conversely, if following application of the first predictive biomarker, the patient has undergone *the TLR9 agonist maintenance immunomodulation regimen* in the previous 3 weeks, then
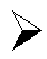 subsequent to being assigned a status of "non-matched-in-second", the TLR9 agonist continues to be administered twice per week, once per week, or every 2 weeks for the subsequent n weeks, wherein n is an integer in the range from 6 to 24. In particular embodiments, n is within the range of 6 to 15 weeks. In more particular embodiments, n is 9 weeks, or
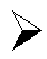 subsequent to being assigned the status of "matched-in-second" (or upon reaching a median threshold value for PFS), the CPM is administered once every 2, or 3 weeks for the subsequent n weeks, wherein n is an integer in the range of 6 to 24 weeks. In particular embodiments, n is within the range from 6 to 12 weeks. In more particular embodiments, n is 9 weeks.

In some embodiments, following repeated application of the second predictive biomarker, treatment switches from administration of a TLR9 agonist 2, 1, a week, or every 2 weeks, to administration a CPM administration every 2, or 3 weeks, or vice versa, once the patient reaches a threshold of a median PFS survival that characterises their cancer disease, for example 188 days PFS for a esSCLC patient, or 225 days PFS for a mCRC patient as demonstrated in the examples, without reference to a second predictive biomarker assessment.

### Predictive Biomarkers

In certain embodiments, the TLR9 agonist, and/or the checkpoint modulator according to any one aspects or embodiments above are administered in a biomarker-led treatment protocol to a patient diagnosed with a type of cancer sensitive to a chemotherapy agent in combination with an immune adjuvant treatment. In particular the cancer is selected from breast cancer, cervical cancer, endometrial cancer, colorectal cancer, metastatic colorectal carcinoma, or lung cancer. In more particular embodiments, the TLR9 agonist, and/or the checkpoint modulator are used to treat small cell lung cancer, particularly extensive stage small cell lung cancer (esSCLC), or metastatic colorectal cancer as studied in the clinical trials analysed in the examples.

In other embodiments of the TLR9 agonist, and/or the checkpoint modulator used according to any one of the preceding aspects or embodiments to treat lung cancer, particularly esSCLC, the patient is assigned a status of "non-matched-in-first" when the result of the first predictive biomarker is below a median threshold value derived from analysis of a cohort of esSCLC patient samples. In particular embodiments, the patient is assigned a status of "non-matched-in-first" when the patient's biomarker value measured in the sample analysed in the first predictive biomarker assay is characterised as:
- a ratio of activated B cells which is about < 16.9% of B cells,
- a ratio of activated T cells which is about < 0.76% of T cells,
- a ratio of Treg cells which is about < 7.6% of CD4+ T cells,
- a ratio of activated NKT cells < 4.1% of NKT cells,
- a ratio of activated pDC is < 7.6% of pDC,
- a ratio of mDC is < 0.48% and/or
- a ratio of activated DC < 54.51% of DC.
invention In particular embodiments, the patient is assigned a status of "non-matched-in-first" when the percent of activated B cells in the patients sample is below a threshold of about < 16.9% of B cells

In alternative embodiments, the patient is assigned a status of "matched-in-first" when the result of the first predictive biomarker is characterised as:
- a percent of activated B cells ≥ 16.9% of B cells, particularly ≥ 21% of B cells,
- a ratio of activated T cells ≥ 0.76% of T cells,
- a ratio of activated Treg cells ≥ 7.6% of Treg cells, and/or
- a ratio of activated NK cells ≥ 4.1% of NK cells,
- a ratio of activated pDC is ≥ 7.6% of pDC,
- a ratio of mDC is ≥ 0.48% and/or
- a ratio of activated DC ≥ 54.51% of DC.

In particular embodiments, the patient is assigned a status of "matched-in-first" when the percent of activated B cells in a patient's PBMC sample is ≥ 16.9% of B cells. In particular embodiments, the patient is assigned a status of "matched-in-first" when the percent of activated B cells in the patient's sample is ≥ 21% of B cells,

In particular embodiments, the percent of B cells expressing the activation marker CD86 is determined in a patient PBMC sample in a first predictive biomarker assay, and the patient is assigned a status of "matched-in-first" when the percent of CD86+ B cells ≥ 16.9% of CD45+ CD19+ B cells. In particular embodiments, the patient is assigned a status of "non-matched-in-first" when the patients sample biomarker value is below threshold of a ratio of activated, CD86+ B cells which is about < 16.9% of total B cells,

In certain embodiments, assignment of a patient to a treatment is carried out by determining the percent of activated immune cells using the following markers quantified by flow cytometry: B cells expressing the marker CD86, activated T cells, and/or NK cells according to the percent which are CD69+, activated pDC, or mDC which are CD40+ or BDCA-2+, particularly BDCA-2+, and/or activated DC which express CD40.

In certain embodiments, the percent of activated cells within B cells, T cells, NKT cells, pDC, or DC is determined in the first predictive biomarker assay.

In certain embodiments, an assay where the percent of activated B cells, and one or more of activated T cells, NKT cells, PDC cells, or DC is determined in the first predictive biomarker assay.

In other embodiments, the percent of activated B cells, T cells, and NKT cells, is determined in the first predictive biomarker assay.

In particular embodiments, the percent of activated B cells is determined in the first predictive biomarker assay.

In certain embodiments, an assay where the percent of B regulatory (Breg) cells is used in the second predictive biomarker assay.

In certain embodiments, activated B cells are defined as CD86+ in in the first, or the second predictive biomarker assay.

In certain embodiments of the invention, an assay where the percent of activated CD86+ B cells and the percent of Treg cells among CD4+ T cells is used in the second predictive biomarker assay.

In particular embodiments of the invention, an assay where the percent of Treg cells among CD4+ T cells is determined, is used in the second predictive biomarker assay.

The median percentage of activated cell subsets which serves as a threshold according to the invention may be calculated from data obtained from analysis of the biomarker expression obtained from a cohort of patient samples as demonstrated in the examples. The cohort of patients are characterised by diagnosis with a cancer derived from the same tissue as the patient being treated, and preferably, having undergone induction chemotherapy at the same timepoint at which the patient undergoes the first, or second predictive biomarker assay, or within 1 to 2 months of the same timepoint. The number of patients in such a cohort is not particularly limited, but should be sufficiently powered to allow discrimination of patients with different outcomes, as demonstrated in the studies of Lefitolimod presented in the examples.

In particular embodiments, the percent of activated immune cells, or the percent of Treg cells is determined in a peripheral blood sample in the first, or second predictive biomarker assay according to the aspects of the invention specified above. In certain embodiments this blood sample is processed, for example by gradient centrifugation, to provide a peripheral blood mononuclear cell (PBMC) sample free of platelets, neutrophils and red blood cells, which is assessed for the presence of immune cell subsets and activation markers, as demonstrated in the examples. In alternative embodiments considered feasible by the inventors, whole blood, or a blood smear, or a histology or biopsy sample is analysed to assess immune activation parameters.

In certain embodiments of the invention, a PBMC sample, or whole blood sample is used in a predictive biomarker assay which has undergone a cell culture step. In this cell culture step, the sample is stimulated with an immune agonist, for example, a TLR9 agonist for a period within the range of 4 to 48 hours. In particular embodiments, a patient PBMC sample stimulated with Lefitolimod for about 24 hours is used in the first, and/or second predictive biomarker assay.

### Induction Chemotherapy

In particular embodiments, the TLR9 agonist, and/or the CPM are used in patients who have received an induction chemotherapy comprising those agents utilised most commonly to treat lung cancer, a platinum-based chemotherapy comprising a platinum-containing drug selected from carboplatin, satraplatin, cisplatin, dicycloplatin, nedaplatin, oxaliplatin, picoplatin, triplatin, and tetranitrate, in combination with etoposide, or topotecan.

The TLR9 agonist, or the checkpoint modulator for use according to the invention may follow different types of induction chemotherapy according to certain embodiments of the invention. In certain embodiments, the induction chemotherapy consists of a first set of treatment cycles comprising 2, 3, 4 or 5 treatment cycles. In particular embodiments, the patient has received 4 sets, or repetitions, of a 3-week cycle of said induction chemotherapy, where a chemotherapeutic agent is administered in the first week of said cycle.

In certain embodiments, the TLR9 agonist, and/or the checkpoint modulator are administered to a patient following an induction chemotherapy period wherein the chemotherapy agent administered comprises an antineoplastic platinum complex drug. In particular embodiments, the antineoplastic platinum complex drug is selected from cisplatin, oxaliplatin, or carboplatin, and is administered together with an antineoplastic alkaloid drug. In particular embodiments, said antineoplastic platinum complex drug is administered in combination with an antineoplastic alkaloid drug selected from etoposide, or topotecan.

In certain embodiments, the induction chemotherapy according the invention is a form of re-induction chemotherapy, where a chemotherapy is administered, or restarted after tumour progression is diagnosed, or recurrence of a tumour occurs after a period of remission. In the IMPALA phase III trial, lefitolimod switch-maintenance therapy had inferior median progression-free survival (mPFS) compared to the control arm. However, in patients receiving re-induction treatment, followed by cycles of chemotherapy and lefitolimod, the mPFS was significantly superior in the chemotherapy/lefitolimod arm compared to control.

In certain embodiments, an antineoplastic chemotherapy drug is administered once every two to four weeks to provide one cycle of the induction chemotherapy. In particular embodiments, the antineoplastic chemotherapy drug is administered once every three weeks of the induction chemotherapy cycle.

Cycles of Induction chemotherapy schemes are typical for chemotherapeutic regimes utilising, for example EGF-, VEGF-specific monoclonal antibody treatment, for example in metastatic breast cancer. In certain embodiments, TLR9 agonists, or immune checkpoint inhibitor are used according to the invention in a patient who has been administered an induction chemotherapy comprising or consisting of intravenous infusion of a hormone receptor-targeted, monoclonal antibody treatment.

In certain embodiments, the TLR9 agonist, and/or CPM are administered to a SCLC, or NSCLC patient following an induction chemotherapy comprising administration of carboplatin or cisplatin in combination with etoposide, or topotecan (as a second line treatment).

**In** certain embodiments the TLR9 agonist, and/or the CPM is administered to a breast cancer, ovarian cancer, or endometrial cancer patient following an induction chemotherapy comprising or consisting of cycles of treatment with carboplatin.

In certain embodiments the TLR9 agonist, and/or the CPM is administered to cervical cancer a patient following an induction chemotherapy comprising or consisting of administration of cycles of treatment with cisplatin.

In certain embodiments, the TLR9 agonist, or the CPM is used in a patient following an induction chemotherapy for colorectal cancer (CRC) comprising or consisting of administration of oxaliplatin. In particular embodiments, the induction chemotherapy comprises administration of oxaliplatin, in a re-induction setting to treat metastatic CRC.

In certain embodiments, the cancer patient receives 6 cycles of treatment with an antineoplastic drug in total, for example, 4 cycles of the antineoplastic drug alone, and 2 cycles of treatment where the antineoplastic drug is used in combination with a TLR9 agonist, or CPM agent in TLR9 agonist, or CPM agent induction immunomodulation treatment cycles.

### Medical treatment, Dosage Forms and Salts

Similarly, within the scope of the present invention is a method or treating cancer in a patient in need thereof, comprising administering to the patient a TLR9 agonist, or CPM, in some cases in combination with an anti-neoplastic chemotherapeutic drug, or drug combinations such as a platinum-based drug combination according to the above description.

Similarly, a dosage form for the prevention or treatment of cancer is provided, comprising a TLR9 agonist molecule, and or a CPM agent according to any of the above aspects or embodiments of the invention.

In certain embodiments of the TLR9 agonist for use according to any of the aspects or embodiments above, the TLR9 agonist is administered by means of subcutaneous injection.

In certain embodiments the TLR9 agonist is administered:
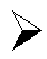 twice per week at a dose of 50-80 mg, particularly 60 mg with a 2, or 3 day interval,
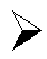 once per week at a dose of 30-60 mg, particularly 60 mg,
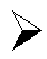 once per week at a dose of 100 to 150 mg, particularly 120 mg, or
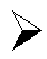 once every 2 weeks at a dose of 100 to 150 mg, particularly 120 mg,

In particular embodiments of the TLR9 agonist for use according to the invention, it is administered once per week at a dose of 60 mg.

In certain embodiments, the checkpoint modulator for use according to the invention is an antibody specific for PD-1 or PDL-1. In particular embodiments, the CPM is an antibody selected from atezolizumab, durvalumab, pembrolizumab, or nivolumab. In more particular embodiments, the CPM is an anti-PD-L1 antibody selected from atezolizumab or durvalumab.

Dosing protocols for checkpoint modulators such as those listed above are well-known for clinical use. CPM are commonly administered by means of intravenous infusion, or by subcutaneous injection, and the dosing may vary depending on the type and severity of cancer, or in response to the occurrence of side-effects. Particular embodiments relate to the use of a CPM delivered by means of an intravenous infusion every three weeks as part of the combined *CPM induction immunomodulation cycle* or single-agent *CPM maintenance immunomodulation regimen.*

The skilled person is aware that any specifically mentioned drug compound mentioned herein may be present as a pharmaceutically acceptable salt of said drug. Pharmaceutically acceptable salts comprise the ionized drug and an oppositely charged counterion. Non-limiting examples of pharmaceutically acceptable anionic salt forms include acetate, benzoate, besylate, bitatrate, bromide, carbonate, chloride, citrate, edetate, edisylate, embonate, estolate, fumarate, gluceptate, gluconate, hydrobromide, hydrochloride, iodide, lactate, lactobionate, malate, maleate, mandelate, mesylate, methyl bromide, methyl sulfate, mucate, napsylate, nitrate, pamoate, phosphate, diphosphate, salicylate, disalicylate, stearate, succinate, sulfate, tartrate, tosylate, triethiodide and valerate. Non-limiting examples of pharmaceutically acceptable cationic salt forms include aluminium, benzathine, calcium, ethylene diamine, lysine, magnesium, meglumine, potassium, procaine, sodium, tromethamine and zinc.

Dosage forms, particularly for chemotherapeutic drugs may be for enteral administration, such as nasal, buccal, rectal, transdermal or oral administration. Alternatively, parenteral administration may be used, particularly for TLR9 agonists or CPM, such as subcutaneous, intravenous, intrahepatic or intramuscular injection forms. Optionally, a pharmaceutically acceptable carrier and/or excipient may be present.

### Pharmaceutical Compositions and Administration

Another aspect of the invention relates to a pharmaceutical composition comprising a compound of the present invention, or a pharmaceutically acceptable salt thereof, and a pharmaceutically acceptable carrier. In further embodiments, the composition comprises at least two pharmaceutically acceptable carriers, such as those described herein.

In certain embodiments of the invention, the compound of the present invention is typically formulated into pharmaceutical dosage forms to provide an easily controllable dosage of the drug and to give the patient an elegant and easily handled product.

The pharmaceutical composition can be formulated for enteral administration, particularly oral administration or rectal administration. In addition, the pharmaceutical compositions of the present invention can be made up in a solid form (including without limitation capsules, tablets, pills, granules, powders or suppositories), or in a liquid form (including without limitation solutions, suspensions or emulsions).

The pharmaceutical composition can be formulated for parenteral administration, for example by i.v. infusion, intradermal, subcutaneous or intramuscular administration.

The dosage regimen for the compounds of the present invention will vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. In certain embodiments, the compounds of the invention may be administered in a single daily dose, or the total daily dosage may be administered in divided doses of two, three, or four times daily.

In certain embodiments, the pharmaceutical composition or combination of the present invention can be in unit dosage of about 1-1000 mg of active ingredient(s) for a subject of about 50-70 kg. The therapeutically effective dosage of a compound, the pharmaceutical composition, or the combinations thereof, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

The pharmaceutical compositions of the present invention can be subjected to conventional pharmaceutical operations such as sterilization and/or can contain conventional inert diluents, lubricating agents, or buffering agents, as well as adjuvants, such as preservatives, stabilizers, wetting agents, emulsifiers and buffers, etc. They may be produced by standard processes, for instance by conventional mixing, granulating, dissolving or lyophilizing processes. Many such procedures and methods for preparing pharmaceutical compositions are known in the art, see for example L. Lachman et al. The Theory and Practice of Industrial Pharmacy, 4th Ed, 2013 (ISBN 8123922892).

### Method of Manufacture and Method of Treatment according to the invention

The invention further encompasses, as an additional aspect, the use of a TLR9 agonist, or CPM as identified herein to treat subsets of cancer patients identified by activated immune cell biomarkers, or its pharmaceutically acceptable salt, according to the treatment protocols as specified in detail above, for use in a method of manufacture of a medicament for the treatment or prevention of cancer.

Similarly, the invention encompasses methods of treatment of a patient having been diagnosed with a cancer disease associated with a susceptibility to immunomodulation, such as TLR9 agonist or immune checkpoint modulation, such a tumour characterised by many mutations, a tumour which has previously responded to a immunomodulation therapy in the past, or which is has been determined to be likely to be sensitive to immunomodulation sue to specific genetic characteristics. This method entails administering to the patient an effective amount of TLR9 agonist or CPM as identified herein, or its pharmaceutically acceptable salt, as specified in detail herein.

The invention further encompasses the use of molecular probes for use assessing the immune cell subset activation markers identified herein, such as antibodies to identify T cells, Tregs, B cells, NKT cells, pDC, mDC, or DC, or their specific activation markers, for use in the manufacture of a kit for the detection of condition cancer in patients that may be amenable to TLR9, or CPM, or alternating TLR9 and CPM treatment according to the biomarker-led treatment protocols specified according to the aspects and the embodiments of the invention provided in the description.

Wherever alternatives for single separable features such as, for example, an cell surface immune cell activation protein or TLR9 agonist, CPM or particular type of cancer are laid out herein as "embodiments", it is to be understood that such alternatives may be combined freely to form discrete embodiments of the invention disclosed herein. Thus, any of the alternative embodiments for a detectable biomarker immune cell population may be combined with any of the alternative embodiments of TLR9 agonist or CPM and these combinations may be combined with any cancer type or diagnostic method mentioned herein.

The invention is further illustrated by the following examples and figures, from which further embodiments and advantages can be drawn. These examples are meant to illustrate the invention but not to limit its scope.

### Description of the Figures

Insets in all Kaplan-Meier plots show hazard ratios with corresponding confidential intervals and log rank p-values.

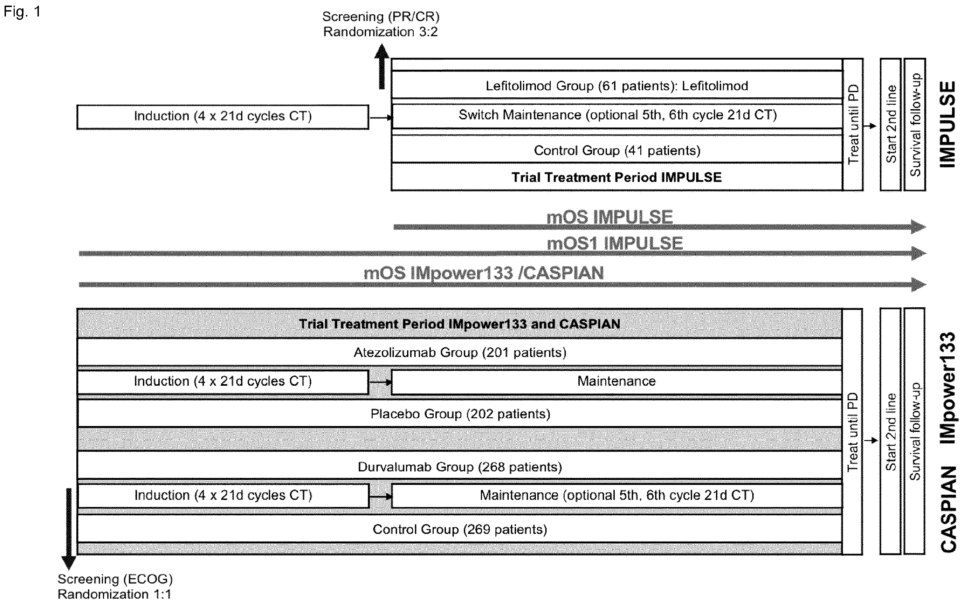

- Fig. 1: shows comparison overlay of IMPULSE progression free survival (PFS) and OS data with studies used for comparative analysis and further representative studies. IMPULSE mPFS1 and mOS1 are calculated from the start of induction chemotherapy. 21d CT: 21-day chemotherapy cycle; ECOG: Eastern Cooperative Oncology Group performance status.

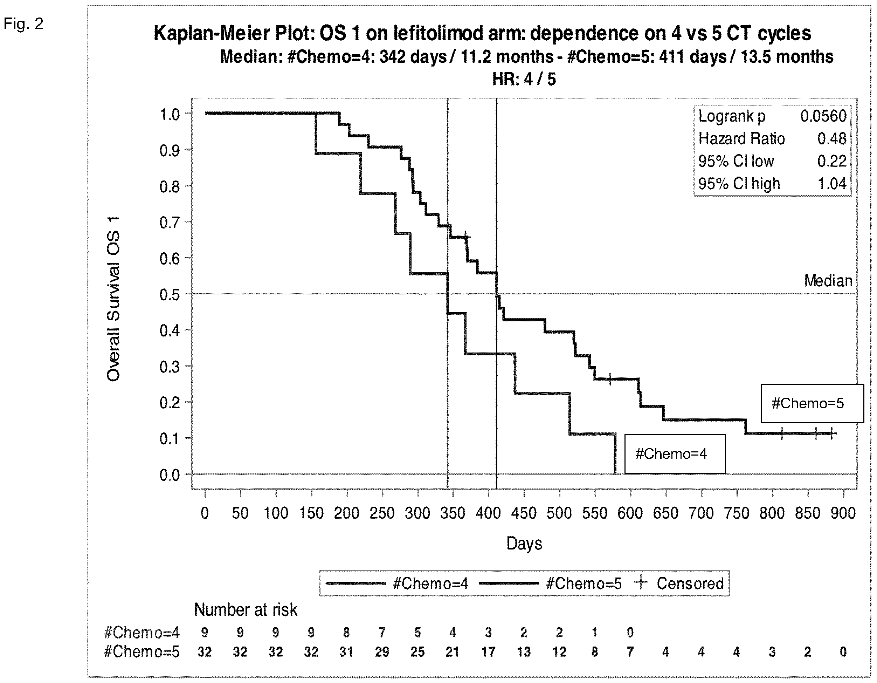

- Fig. 2: shows a comparison of overall survival (OS1) of IMPULSE patients with 4 cycles, or 5 cycles of PE chemotherapy followed by subsequent treatment with TLR9 agonist. The gain in mOS is 2.3 m, and a plateau region typically observed with immunotherapies becomes visible. #Chemo=5, patients treated with a fifth cycle of PE chemotherapy, or #Chemo=4, patients treated with four cycles PE induction chemotherapy.

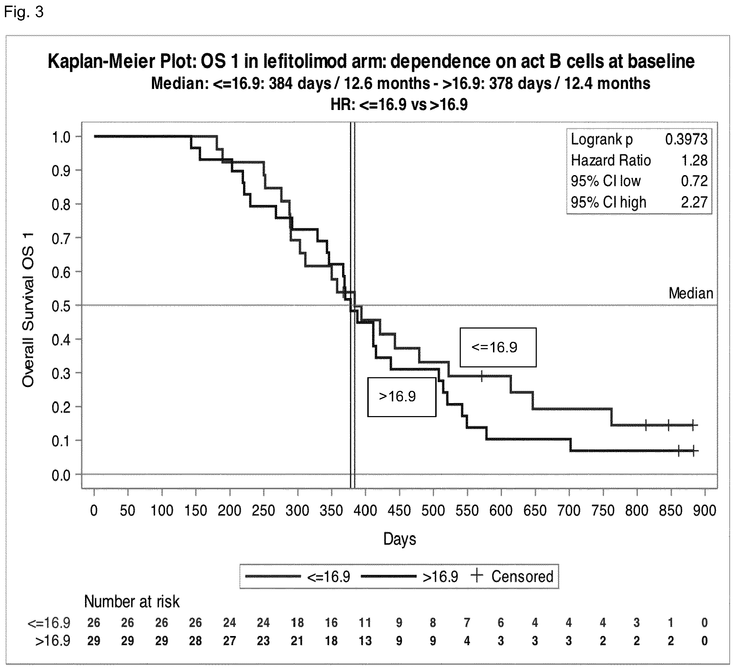

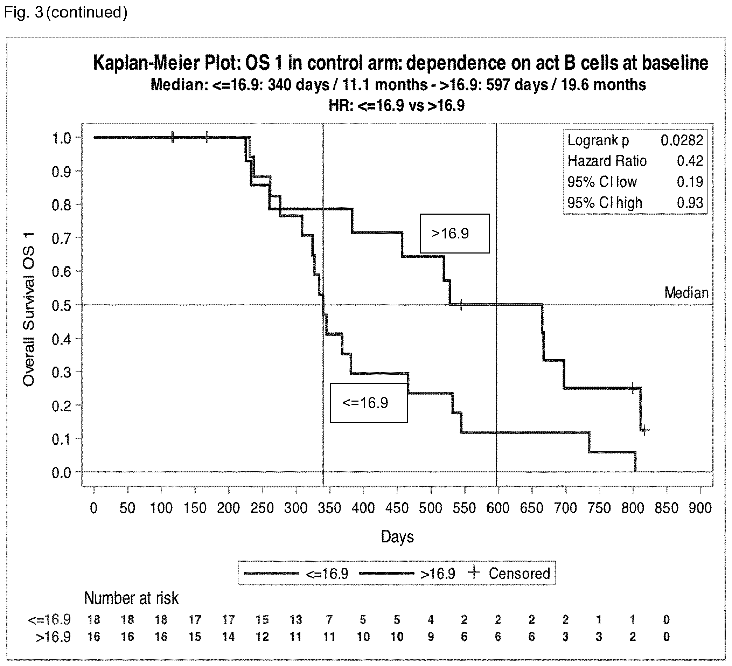

- Fig. 3: shows Kaplan-Meier plots comparing OS1 for IMPULSE lefitolimod patients, or control arm (continuation maintenance therapy) according to B cell activation status, the percentage of CD45+CD19+ B cells positive for CD86. Cell activation was measured by flowcytometry in baseline peripheral blood mononuclear cell samples following 24h culture with MGN1703 (lefitolimod). Samples are dividing below or equal to the median (≤), or above the median (>) percent of activated cells of each indicated population.

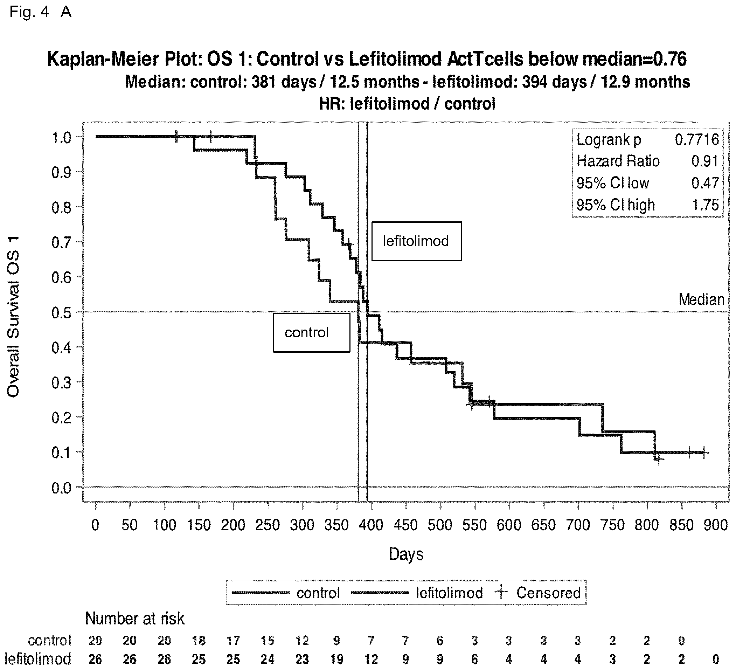

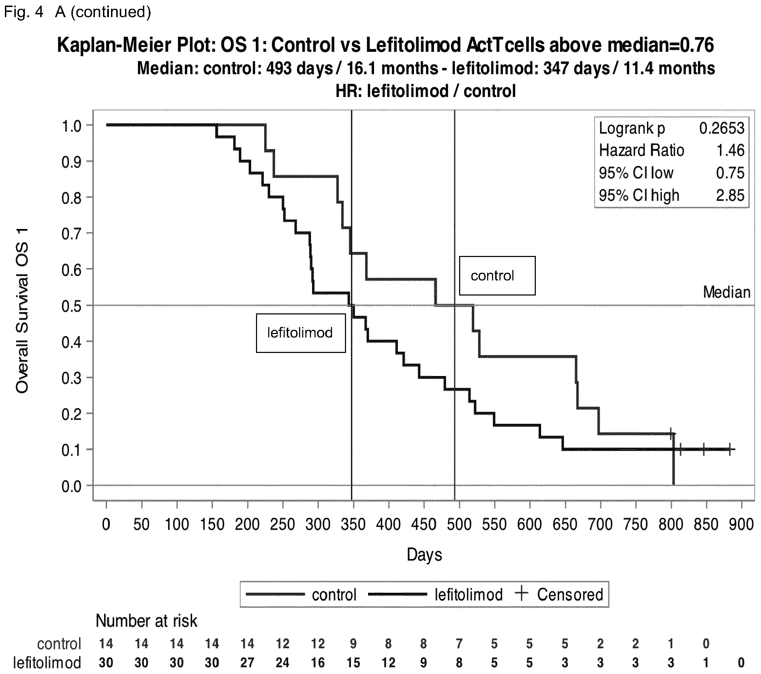

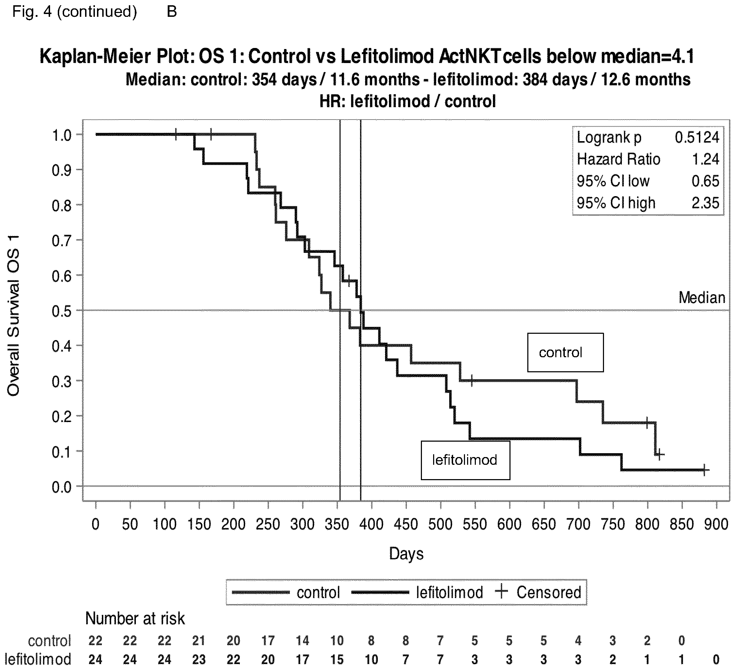

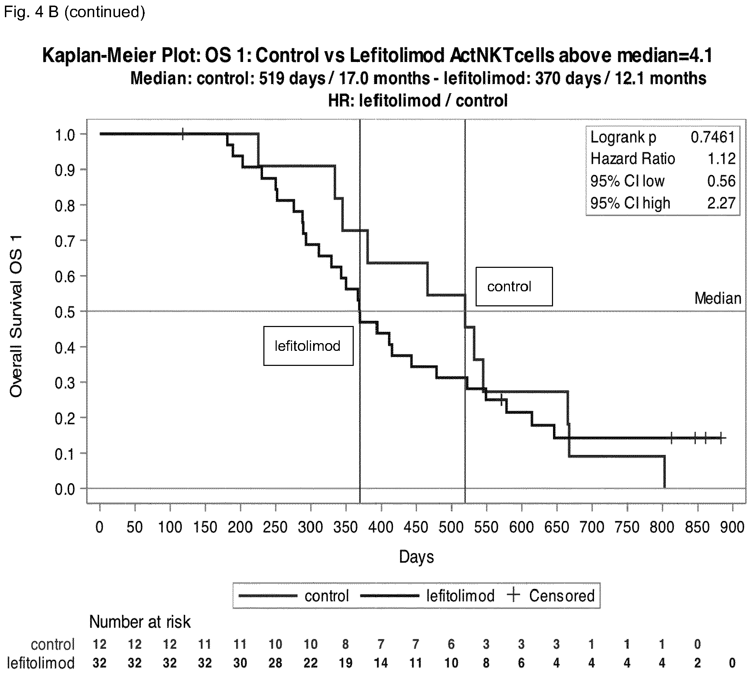

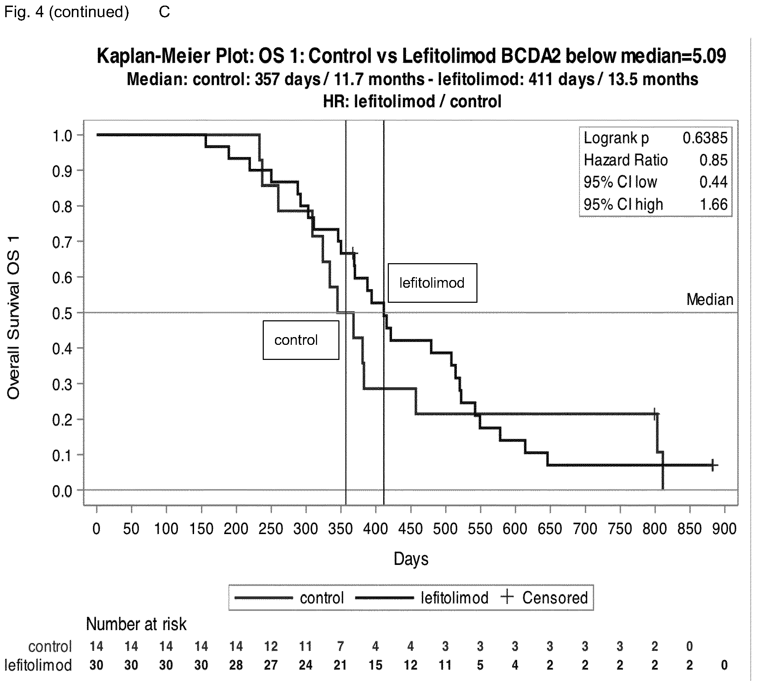

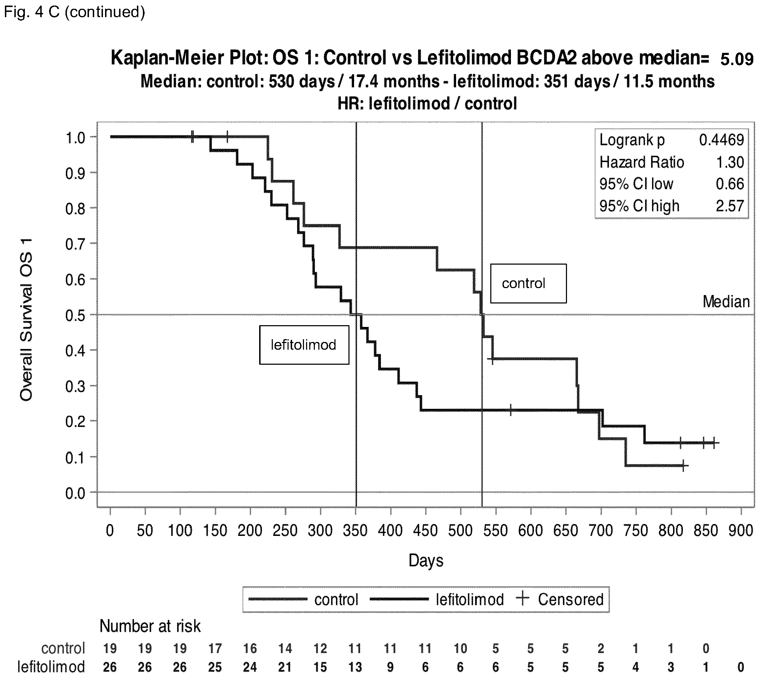

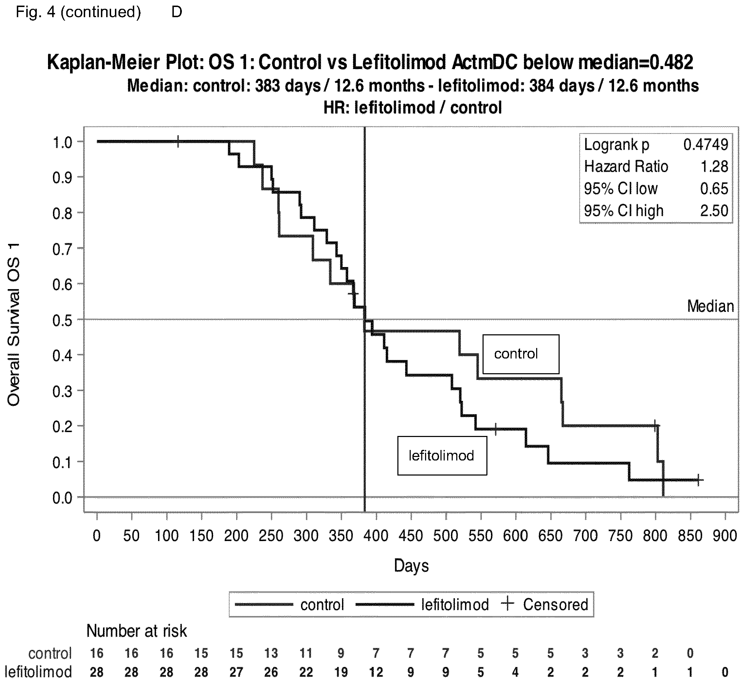

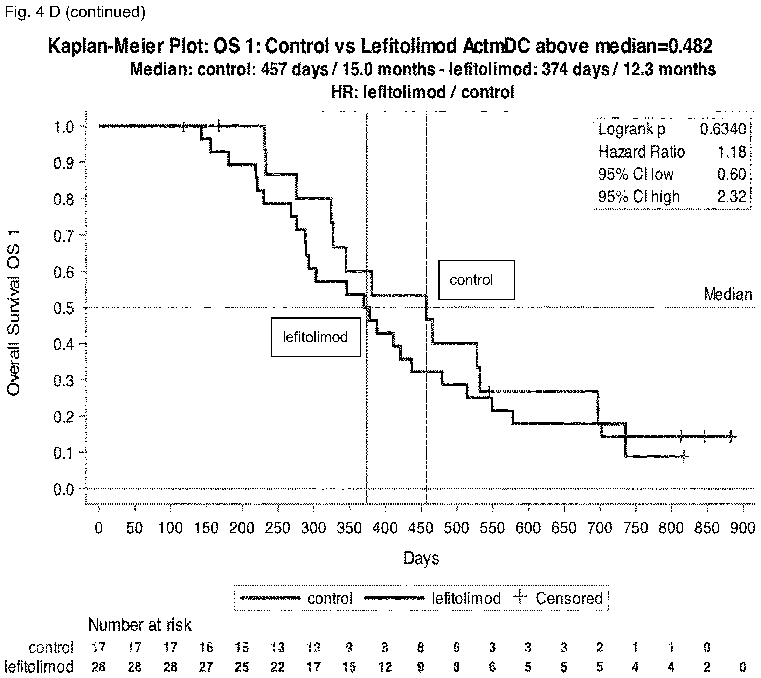

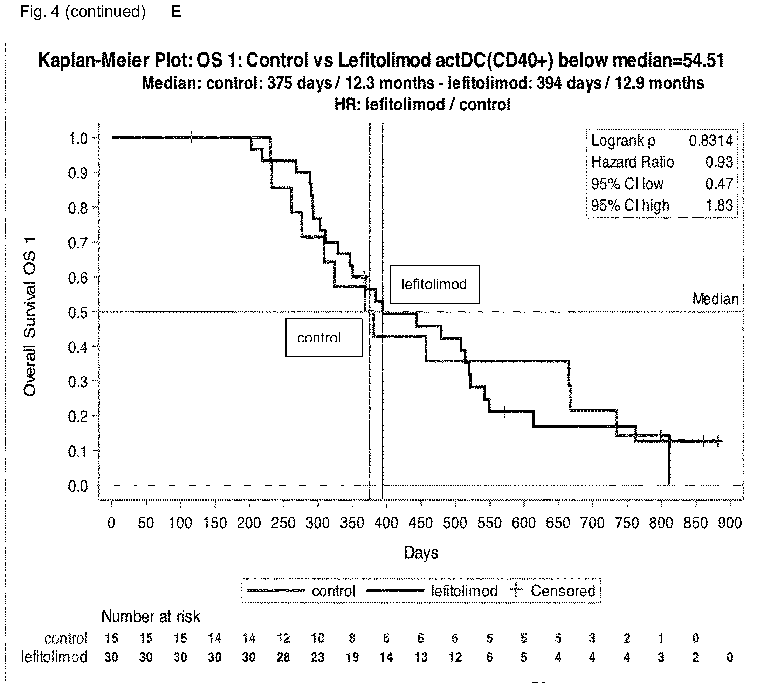

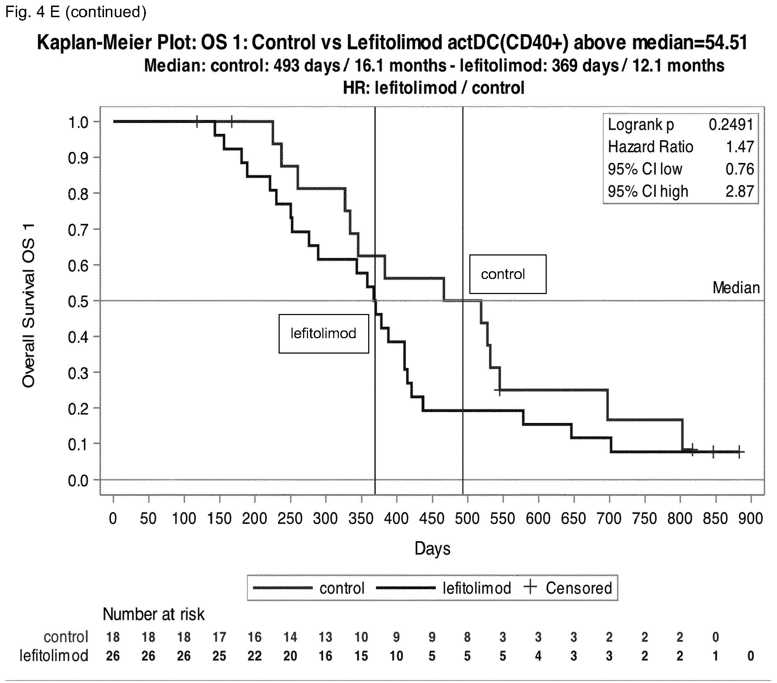

- Fig. 4: shows Kaplan-Meier plots comparing OS1 for IMPULSE patients receiving lefitolimod switch maintenance, or control arm treatment (continuation maintenance therapy), separated according to activated immune cell biomarker status ≤ (left), or > (right), a median threshold value of:
A. T cell activation status, percentage of CD45+ CD3+ T cells positive for CD69+,
B. Natural killer T (NKT) cell activation status, percentage of CD45+ CD3+ CD56+ T cells positive for CD69,
C. Plasmacytoid dendritic cell (PDC) activation status, percentage of lineage (CD3/CD14/CD16/CD19/CD20/CD56)- CD45+ CD123+HLA-DR+ PDC positive for BCDA2,
D. Myeloid dendritic cell (mDC) activation status, percentage of CD45+ lineage- CD11c+HLA-DR+ myeloid dendritic cells positive for CD86,
E. Dendritic cell (DC) activation status, percentage of CD45+ lineage- HLA-DR+ myeloid dendritic cells positive for CD40.
- Fig. 5: shows determination of an optimal progression free survival (PFS) biomarker threshold. Left panel, ROC curve, right panel, Youden index. AUC, area under curve. A larger AUC indicates improved outcome in the receiver operating curve (ROC) while in the Youden index, a higher the point of intersection of the two curves indicates optimal biomarker performance.

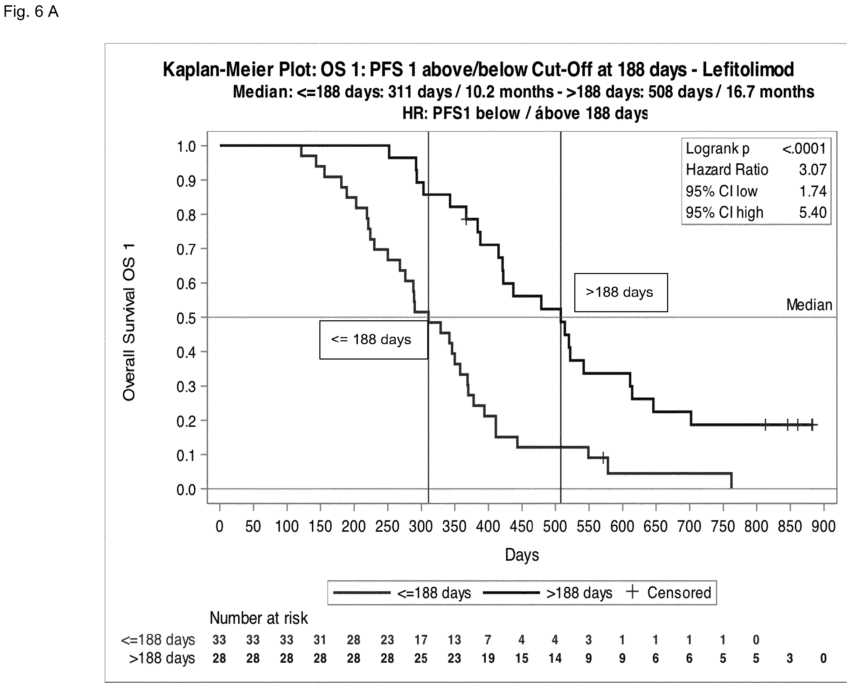

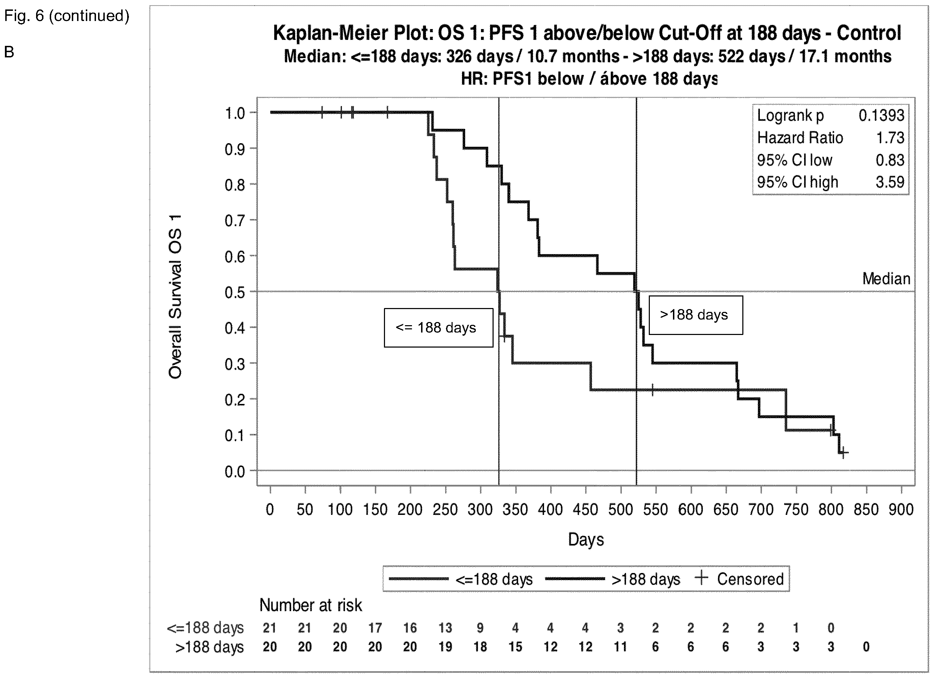

- Fig. 6: shows Kaplan-Meier plots comparing IMPULSE patients who had PFS1 above a threshold of 188 days with patients who had PFS below threshold of 188 days. A, switch maintenance therapy with TLR9 agonist, B, PE chemotherapy as continuation maintenance therapy, according to PFS1 > or ≤188 days.

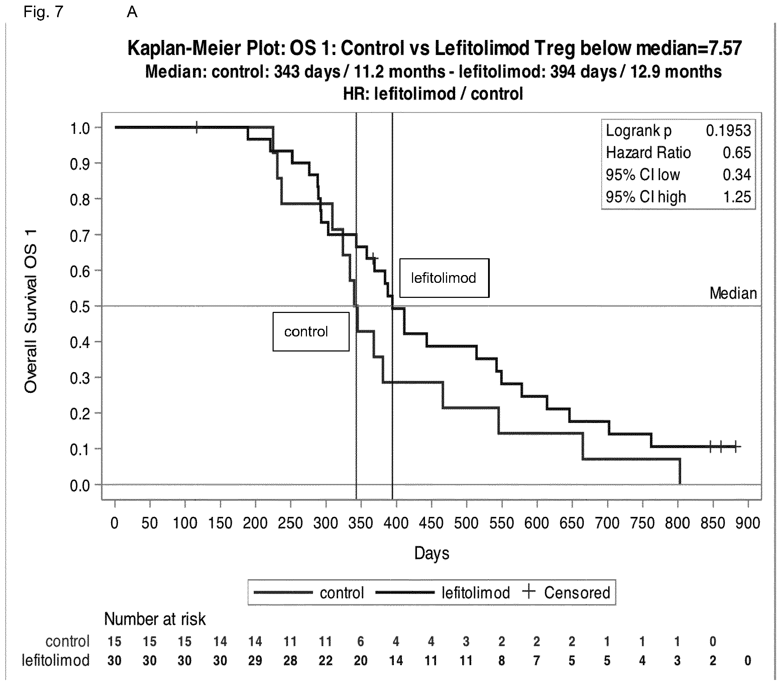

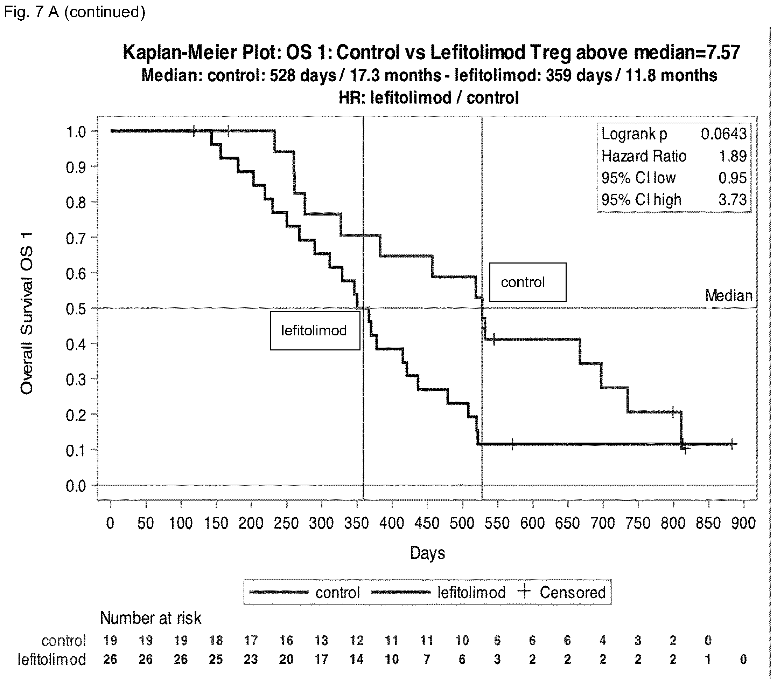

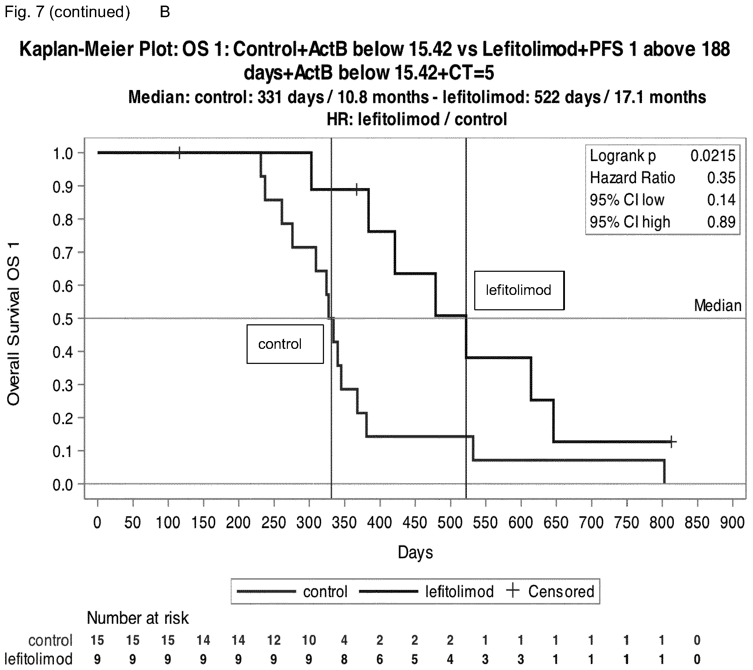

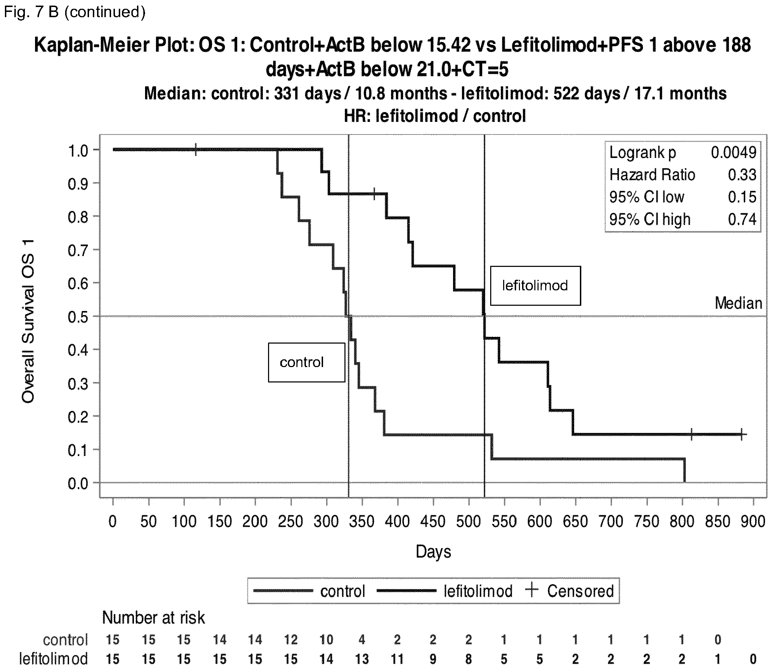

- Fig. 7: A. shows Kaplan-Meier plots comparing IMPULSE patients receiving switch maintenance therapy with TLR9 agonist or control continuation maintenance therapy according to whether the percentage of CD45+ CD3+ CD4+ CD25+ CD127- T regulatory (Treg) among gated CD45+ CD3+ CD4+ T cells was > or ≤ the median value. B. shows Kaplan-Meier plots comparing IMPULSE patients receiving switch maintenance therapy with TLR9 agonist or control, standard of care continuation maintenance therapy split according to either ≤median 16.9% or ≤ 21% percent pf CD86+ activated B cells after induction or re-induction chemotherapy. Patients with activated B cells ≤median (16.9%) after induction or re-induction chemotherapy, treated with TLR9 agonist switch maintenance in sequence following a 5^{th} cycle of PE chemotherapy, and having a PFS1 >6.2 months with patients in continuation maintenance with activated B cells ≤ median (16.9 %, or ≤ 21%) after induction or re-induction chemotherapy, treated with 4, 5, or 6 cycles of PE chemotherapy.

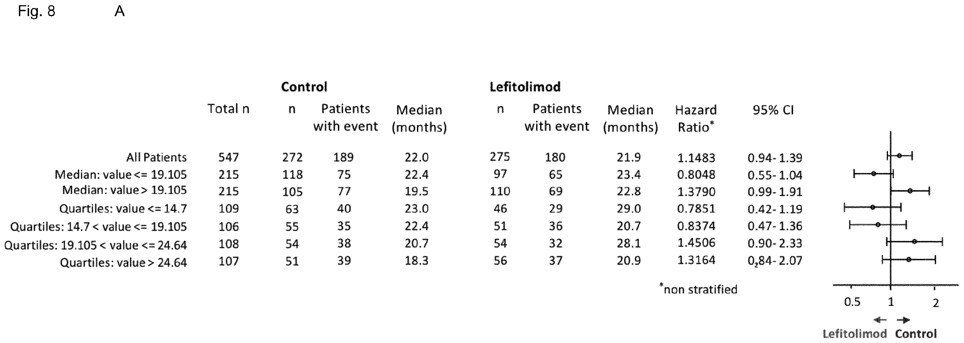

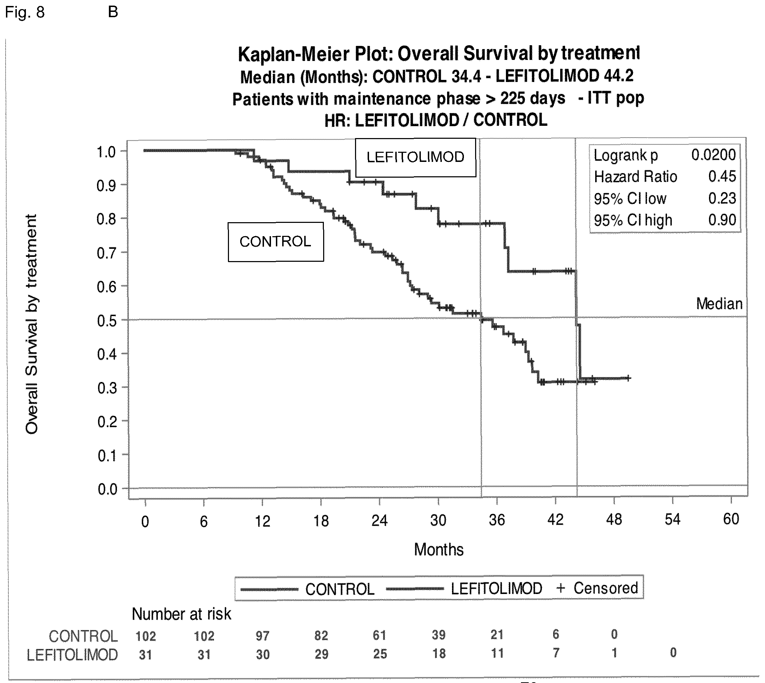

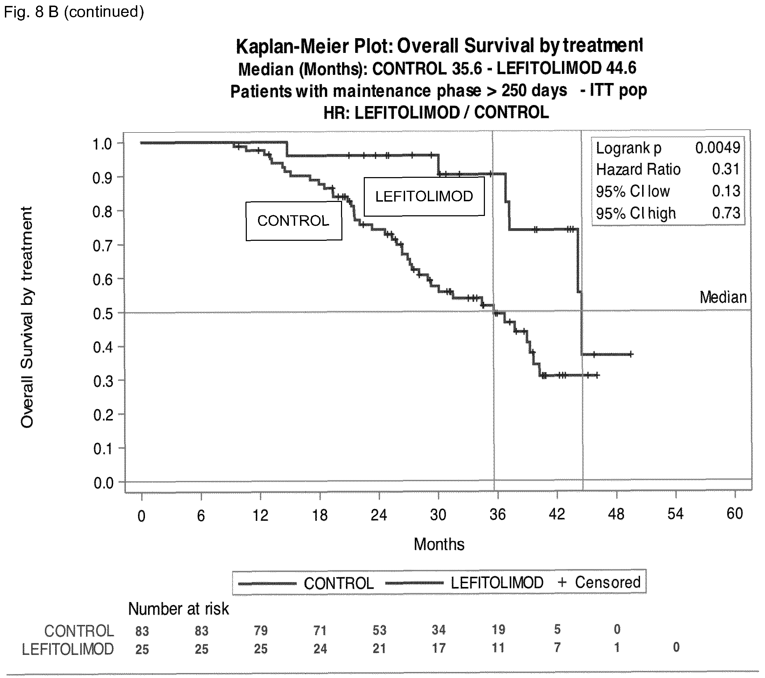

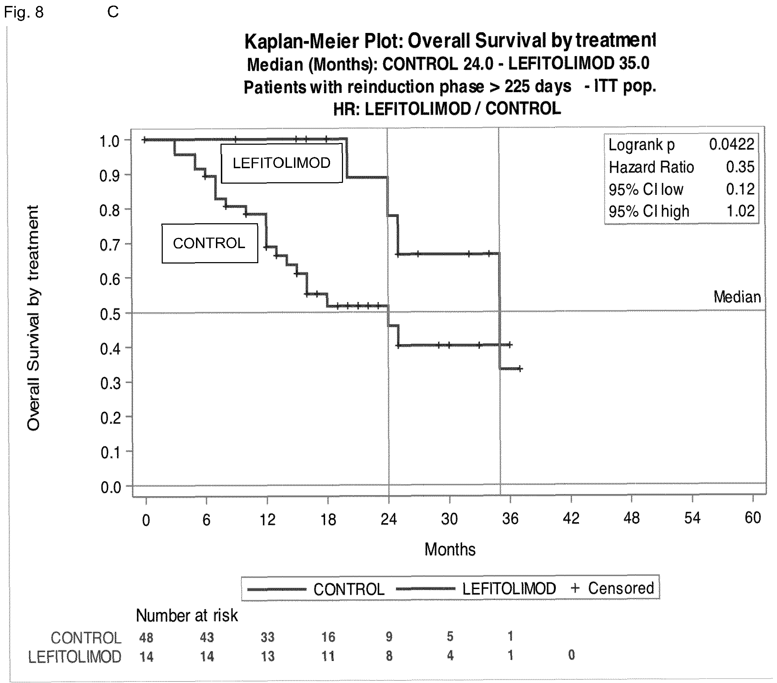

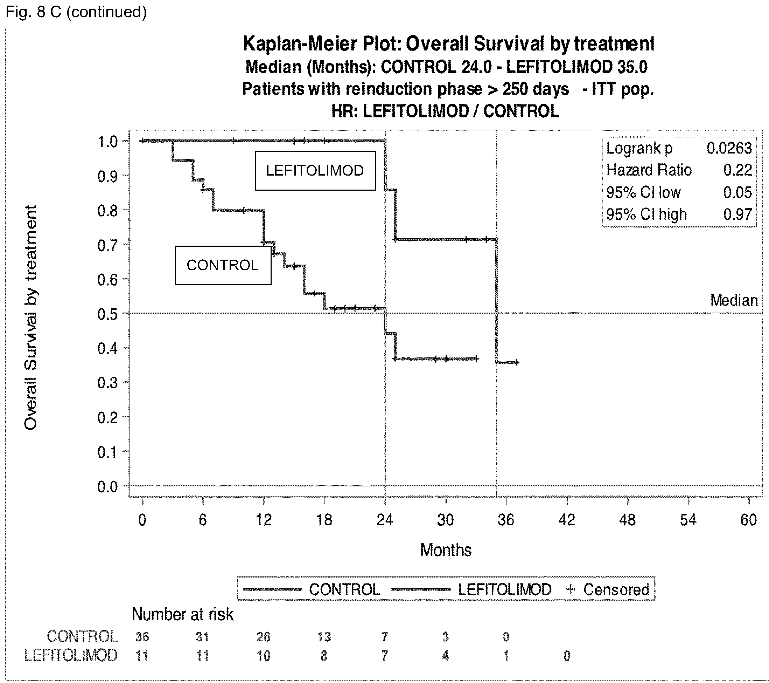

- Fig. 8: shows second biomarker components analysed in patients from the IMPALA metastatic colorectal cancer trial treated with lefitolimod or control standard of care. A: activated B cell biomarker values and relative distribution of the percent of CD45+CD19+ B cells positive for CD86 in the indicated treatment arms. Kaplan-Meier plots comparing overall survival (OS) of IMPALA patients receiving TLR9 agonist or control continuation maintenance therapy, split according to B. progression free survival > 225 days, or > 250 days of maintenance phase treatment, C. progression free survival > 225 days, or > 250 days of reinduction phase treatment.

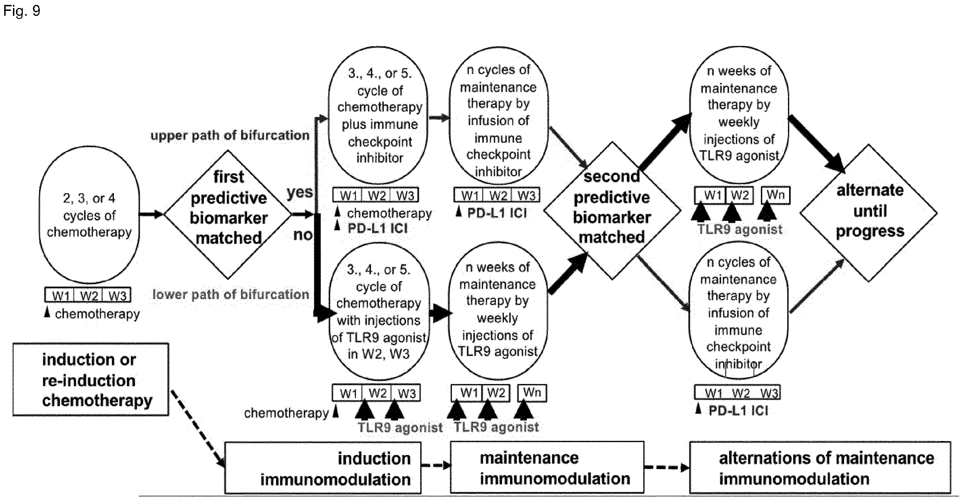

- Fig. 9: shows a sequential, two-step, biomarker-guided, bifurcated, alternation of innate and adaptive immunomodulation model to follow induction (for example platinum-etoposide induction chemotherapy for esSCLC, IMPULSE) or re-induction chemotherapy-based treatment for cancer (5-FU/FA, or CAPE, plus OX, or IRI, alone or with anti-VEGF or anti-EGFR antibodies for mCRC, IMPALA). This model is projected to have to most efficacious effect due to the tailored application of innate or adaptive immune stimuli based on median biomarkers reflecting patient adaptive immune activation levels.

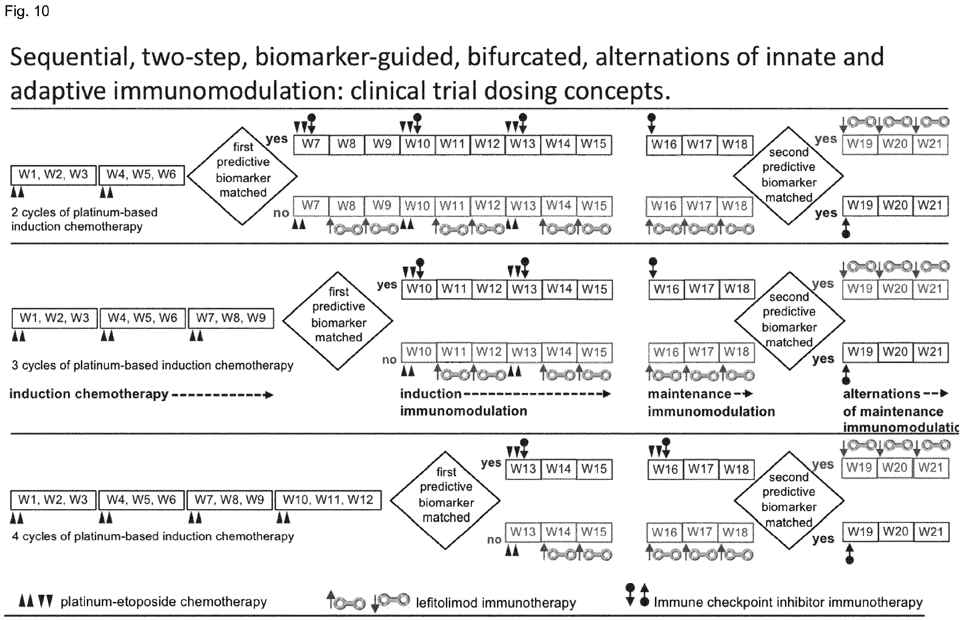

- Fig. 10: shows representative examples of immunomodulation treatment regimens based on varying lengths of platinum-based induction chemotherapy treatment.

### Examples, Methods

### IMPULSE study, NCT02200081

An international, multicentre, open-label phase II trial conducted on 102 extensive stage small cell lung cancer after platinum based first line therapy patients. Patients who had received four cycles of first line induction chemotherapy (platinum-based) were randomized to receive either combination lefitolimod maintenance therapy, or local standard of care (control). After progression, patients received second line therapy.

### IMPALA study, NCT02077868

An international, multicentre, open-label phase III trial conducted on 549 metastatic colorectal cancer patients in 8 EU countries. Patients who had an objective response to any first line induction chemotherapy (5-FU/FA, or CAPE, plus OX, or IRI, alone or with anti-VEGF or anti-EGFR antibodies) were randomized to receive either lefitolimod monotherapy, or local standard of care (control). After progression, patients started re-induction therapy, with those in the experimental arm continuing to receive lefitolimod in addition.

### Cell Culture in Vitro Biomarker Test

Approximately 20mL whole blood collected in lithium-heparin tubes on scheduled visits, was analyzed no later than 24 hours after collection and transported at room temperature (18°C to 24°C). Peripheral blood mononuclear cells were isolated by density gradient centrifugation (Ficoll). To determine the individual response of each patient to lefitolimod MGN1703 stimulation in vitro, cytokine and chemokine production of PBMC were analyzed. PBMC were treated with MGN1703 for 24 hours. Activation of immune cells was measured by flow cytometry, membrane-bound proteins were stained on intact cells with monoclonal antibodies in phosphate-buffered saline containing 10% (v/v) human serum, 2.5% (v/v) foetal calf serum, and 0.1% (w/v) azide on ice. The following antibodies were used: anti-lineage cocktail 1*CD3, CD14, CD16, CD9, CD20, CD56), anti-CD123 (7G3), anti-HLA- DR (L243), anti-CD40 (5C3), anti-CD11c (B-ly6), anti-CD86 (2331 FUN-1), anti-CD19 (4G7), anti-CD69 (FN50), all from BD Biosciences; anti-CD14 (61D3), anti-CD169 (7-239), anti-CD3 (OKT-3), anti-CD56 (MEM188), anti-CD80 (2D10), all from eBioscience.

### Example 1: Sequential, two-step, biomarker-guided, bifurcated alternations of innate and adaptive immunomodulation, following induction or-reinduction chemotherapy.

The phase II IMPULSE study explored the benefit in OS of an immunotherapeutic switch maintenance therapy of ES-SCLC by the TLR9 agonist lefitolimod (dSLIM-30L1). In comparison to the various local standards of care (SoC), typically carboplatin plus etoposide, or cisplatin plus etoposide. No additional benefit was observed from lefitolimod switch maintenance therapy reached as assessed by median progression-free survival (mPFS), or median overall survival (mOS). However, certain subgroups of patients showed strong median OS improvements and the biological mode-of-action, i.e., immune surveillance reactivation, confirmed by analysis of immunogenicity in patient samples (Thomas M. *et al.,* 2018 *Ann. Oncol.* **29**(10):2076). A combinatorial post hoc analyses was performed of all accessible immunological data and relevant clinical parameters of the IMPLUSE study. Additionally, in order to determine in what conditions adaptive or innate immune stimulation may enhance chemotherapy outcomes, published mPFS and mOS data from the respective treatment and control arms for chemotherapy immunomodulation maintenance studies utilizing PD-L1 inhibitor-antibodies were incorporated, comparing the use of checkpoint inhibitors with the TLR9 agonist used in IMPULSE.

Lefitolimod immunomodulation maintenance therapy was compared to IMpower133 (Horn L. et al., 2018 N. Engl. J. Med. 379:2220), (ClinicalTrials.gov Identifier: NCT02763579, Atezolizumab) and CASPIAN (Paz-Ares L. et al., 2019 Lancet 394(10212):1929), (ClinicalTrials.gov Identifier: NCT03043872, Durvalumab). Outcomes from the Keynote-604 (Rudin C. et al., 2020 J. Clin. Oncol. 38:9001), (ClinicalTrials.gov Identifier: NCT03066778, Pembrolizumab), and EA5161 (Leal T. et al., 2020, J. Clin. Oncol. 38:9000), (ClinicalTrials.gov Identifier: NCT03382561, Nivolumab) studies were considered as comparators.

All lung cancer trials used (IMPULSE, IMpower133, CASPIAN, Keynote-604, and EA5161) are "maintenance" trials, wherein an immune induction treatment, a set of chemotherapy cycles, is applied to initiate anti-tumour responses (as defined by RECIST guidelines), followed by maintenance therapy until tumour progress. IMpower133, CASPIAN, Keynote-604, and EA5161 are "continuation maintenance" trials, whereas IMPULSE is a "switch maintenance" trial. In the four continuation maintenance trials, the immunomodulatory product (IMP), i.e., the respective PD-L1 checkpoint inhibitors (CPI) atezolizumab, durvalumab, pembrolizumab, or nivolumab, are present during induction chemotherapy and in the maintenance phase. In the switch maintenance trial IMPULSE, the IMP lefitolimod is not used during induction chemotherapy but introduced ("switched" to) in the maintenance phase of the trial (Fig. 1). Thus, for comparison of IMPULSE PFS and OS data with the other studies, IMPULSE mPFS1 and mOS1 were factored from the start of induction chemotherapy. Therefore, for all following analyses, the respective mOS1 values of IMPULSE are used.

Table 1 offers a comparison of mOS of IMPULSE with the respective treatment and control arms of IMpower133, CASPIAN, Keynote-604, and EA5161. Evidently, the mOS reached by IMPULSE, IMpower133, and CASPIAN is similarly high, whereas mOS of Keynote-604 and EA5161 is lower by several months. The mOS reached by the pivotal phase III trials IMpower133 and CASPIAN led to the approval of atezolizumab (Tecentriq^{®} by Roche) and durvalumab (Imfinzi^{®} by AstraZeneca). The differing definition of clinical success (IMpower133 and CASPIAN) or failure (IMPULSE) is relative to the mOS of the control arms of each respective study. The control arm mOS of IMpower133 is 10.3 months (m), the corresponding mOS of CASPIAN is also 10.3 m. Both trials' mOS come close to previously mOS for these patients of 10.7 m (Steffens C. C. et al., 2019 Lung Cancer 130:216) and were therefore considered comparable and combined with the IMPLUSE data for the purpose of this study.

### I. Advantages of a consecutive immunomodulation strategy

Analysis of lefitolimod treatment in induction chemotherapy (as a first-line treatment of esSCLC, IMPULSE) compared to a re-induction chemotherapy (as in first-line metastatic colorectal carcinoma (mCRC)) and immunomodulation by TLR9 agonists, IMPALA, Fig. 8) shows the TLR9 agonist is more efficacious in terms of mOS, when applied subsequent to a platinum-based chemotherapy, rather than in combination.

**Example:** two, three or four qw3 cycles of platinum/etoposide (PE) chemotherapy (first cycle, PE chemotherapy applied in respective first week, no therapy in respective second and third week; second cycle, and so on) are sequentially followed by qw3 cycles of PE chemotherapy, where PE is applied in the respective first week of a cycle, and a TLR9 agonist in the respective second and third week of a cycle.

**Evidence:** if four qw3 cycles of PE chemotherapy are sequentially followed by a 5^{th} cycle of PE chemotherapy, where PE chemotherapy in the first week of the cycle is followed by TLR9 agonist application in the second and third week of the cycle, mOS of patients is improved by 2.3 months (Fig. 2).

### II. Predictive biomarker analysis for optimal induction immunomodulation

After the first, second, or third qw3 cycle of induction, or re-induction chemotherapy, and prior to the qw3 cycles with sequential additions of TLR9 agonist treatment, a collection of predictive biomarkers was determined. In order to assess the utility of a biomarker threshold with possible application to cancers derived from various tissues, overall survival was analysed in patient groups divided according to a threshold of the median proportion of activated immune cells of samples from the cohort of cancer patients. Those patients with activated immune cells equal to, or below the median threshold exhibit little evidence of adaptive immune induction, those above are characterized by the terms "activated immune cells above median" or "activated immune cells^{high}", indicating that the respective fractions of activated B cells, activated T cells, activated regulatory T cells, or activated NKT cells, dendritic cells or plasmacytoid dendritic cells are above the median value determined for the cohort of patients having gone the same time since onset of induction chemotherapy, and having being diagnosed with a cancer derived from the same tissue of origin.

**Example:** extensive stage small-cell lung cancer (esSCLC).
- fraction of activated B cells above a median of 16.9%
- fraction of activated T cells above a median of 0.76%
- fraction of activated regulatory T cells above a median of 7.6%
- fraction of activated natural killer T cells above a median of 4.1%,
- fraction of activated pDC is < 5.1% of pDC,
- fraction of activated mDC is < 0.48 % and/or
- fraction of activated DC < 54.51% of DC.

**Evidence:** Patients with activated immune cells above the cohort median show increased overall survival. For example, B cells above median (>16.9%), who received PE chemotherapy as continuation maintenance, have a mOS of 17.9 months, whereas patients with activated B cells below median (≤16.9% have a mOS of just 10.8 months (Fig. 3). Similar results were observed with further immune subsets (Fig. 4).

In the proposed treatment model design sensitive to these groupings, patients matching the predictive biomarkers **"activated immune cells^{high}"**, of which activated B cells offer the statistically most significant value, are assigned to the "upper path of bifurcation", comprising a period of checkpoint inhibition. Patients not matching **"activated immune cells^{high}"**, are assigned to the "lower path of bifurcation" comprising TLR9 agonist treatment (Fig. 9 and 10).

### III. Predictive biomarker analysis for optimal maintenance immunomodulation

SCLC patients reaching a certain threshold in progression-free survival (PFS) benefit are likely to exhibit a significant gain in overall survival (OS). Methods of choice for defining an optimal threshold are the "Youden index" in combination with "receiver operating characteristic curves" (ROC curves). Of the many Youden indices and ROC curves compared, the pair of curves shown in Fig. 5 represents the optimal threshold.

**Example:** esSCLC patients who reach a PFS1 of >188 days (>6.2 months) will most likely experience an OS1 of more than 350 days (11.5 months). This optimum threshold closely resembles the PFS1 values found for the respective full analysis sets (FAS) of patients under switch maintenance therapy by TLR9 agonist (6.1 months) and under PE chemotherapy as continuation maintenance therapy (6.7 months).

**Evidence:** If the above derived PFS threshold of >6.2 months is used in Kaplan-Meier plots to calculate mOS1 for the patient subgroups under switch maintenance therapy by TLR9 agonist lefitolimod and under continuation maintenance therapy, respectively, 17.1 months and 16.7 months were measured (see Fig. 6). The corresponding mOS1 values for a PFS threshold ≤6.2 months reach 10.7 months and 10.2 months, only, matching mOS found in control arms of IMpower133 and CASPIAN trials (10.3 months).

The fraction of regulatory T cells (Treg) increases from start of "induction immunomodulation" (baseline, days 90-104 after start of "induction chemotherapy") followed by "maintenance immunomodulation" (visit 2, days 118-125; visit 3, days 146-153; visit 4 days 188-195) by ≥ 20%. Visit 4 corresponds to the optimum PFS threshold statistically determined (see and Table 2). This yields an absolute measurement for the "second predictive biomarker". It is matched if the fraction of Treg of a patient is ≥20% at approximately ≥6months, 188 days after start of "induction chemotherapy" (Fig. 7A). Similarly, at later timepoints activated B cells above a median corresponding to the optimum PFS threshold may also be used to identify patients with a significant gain in overall survival (Fig. 7B).

### Targeted treatment algorithms for cancer immunomodulation

Results of the deep combinatorial post hoc analyses of clinical trial data shown under I, II, and III establish the essential components of a treatment algorithm for first-line induction or re-induction (platinum-based) chemotherapies. The essential components are:
1) "sequential",
2) "two-step, biomarker-guided, bifurcated", and
3) "alternations of innate and adaptive immunomodulation" .

1) "Sequential" refers to the adoption of a natural sequence of activating triggers in the pathways of innate and adaptive immunity. In all approved immune-oncological treatment protocols, the respective immunotherapeutic medicines are "combined" with chemotherapy or targeted therapies, i.e., applied together, or on the same day. For example, in first line therapies of esSCLC in the trials analysed in this study, PE chemotherapy is given together with PD-L1 inhibiting antibodies atezolizumab (Tecentriq^{®}, Roche) or durvalumab (Imfinzi^{®}, AstraZenenca).

Th1 immunity is generated by chemotherapy, in addition the data above demonstrate the existence of two patient subgroups following PE "induction chemotherapy", i.e., patients with activated immune cells above median, "activated immune cells^{high}" (preferentially activated B cells, but also activated T cells, activated regulatory T cells, and activated NKT cells) after 4 cycles of PE "induction chemotherapy", and patients with activated immune cells below median, "activated immune cells^{low}". Notably, this distinction only arises after several cycles of "induction chemotherapy" generate the differing immune response which may differentiate the two patient populations, hence combinations with CPM agents such as immune checkpoint inhibitor (ICI) or TLR-9 agonists from the onset "induction chemotherapy" are not justified by immune readouts. This however is the current standard of care (SoC) based on the highly similar outcomes of IMpower-133 and CASPIAN clinical studies (Fig. 1 and Table 1).

The post hoc analysis of the IMPULSE and IMPALA clinical trials herein suggests that an immune response must first be generated by "induction chemotherapy" (4 cycles of PE "induction chemotherapy" in IMPULSE), before the natural down-regulatory reactions can be efficaciously counteracted by application of ICI targeting adaptive immunity (such as PD-L1 blocking). The proposed treatment algorithm therefore starts with 2, 3, 4, or 5 cycles of a platinum-based "induction chemotherapy" (shown as "induction or re-induction chemotherapy" in Fig. 9 and Fig. 10), before matching of predictive biomarkers is determined, and the respective sequential treatment commences ("induction immunomodulation").

The forecast transition from "maintenance immunomodulation" to "alternations of maintenance immunomodulation" is also sequential. Here, "sequential alternations" of either treatment with TLR9 agonist or treatment with ICI are offered as an alternative to "combinations" of both an adaptive and immune stimulant at the same time. Further rationale for the "alternations" is explained under 3).

2) "two-step, biomarker-guided, bifurcated" refers to the assessment of predictive immune biomarkers allow selection of patients for a therapy program associated with the best available clinical outcome. In the "bifurcated pathway" approach here, matching or not matching the first biomarker will not exclude patients from treatment, but assign them to either the upper or the lower pathway (Fig. 9 and Fig. 10). "Two-step" refers to the two biomarkers applied, one to select an appropriate induction immunomodulation agent, and a second to guide immunomodulation maintenance therapy, or in some cases, to guide the timing of alternating therapies. Matching or not matching the second biomarker does not exclude patients from treatment but assigns them a timeframe for the alternations in either TLR9 (innate) or ICI (adaptive) maintenance immunomodulation.

### First predictive biomarker:

- fraction of activated B cells above median (Fig. 3)
- fraction of activated T cells above median (Fig 4)
- fraction of activated regulatory T cells (Treg) above median (Fig 4)
- fraction of activated natural killer T cells above median (Fig 4)
- fraction of activated dendritic cells above median (Fig 4)
   fraction of activated myeloid dendritic cells above median
- fraction of activated plasmacytoid dendritic cells above median (Fig 4).

### Second predictive biomarker:

- progression-free survival (PFS) > median of 188 days, 6.2 months (Fig 5 and 6)
- fraction of activated regulatory B cells (Treg) ≥ median after >6 month from start of induction chemotherapy (Fig. 7)
- fraction of activated regulatory T cells (Treg) ≥20% after >6 month from start of induction chemotherapy (preferred criterion) (Fig. 7).

3) **"alternations of innate and adaptive immunomodulation"** is another key concept of the treatment algorithm presented herein. In all the many "combination" approaches of immunomodulators (for example, TLR agonists, cGAS-STING, RIG-I) with immune checkpoint inhibitors (PD-1, PD-L1 blockers are regulatory approved examples), time course and quality of immune responses to tumour therapies are not taken into account, although the qualities and quantities of immune cells as well as chemo- and cytokines analysed by "liquid biopsies" are able to reflect the immunobiological situations in the tumour micro-environment (TME).

Patients with liquid biopsy sample measurements classified as activated immune cells^{high} (or above median) following induction or re-induction chemotherapy, likely reflect immunologically "hot" but "immune edited" tumour micro-environments (TME). Here, the inventors predict that treatment with a TLR9 agonist is likely to increase the fraction of mistargeted, activated B cells, exhausted T cells, and regulatory cells (Breg and Treg). These patients, correlating with those matching the first predictive biomarker, are assigned to the upper path of the bifurcation (Fig. 9 and Fig. 10). Their "induction immunomodulation" consists of platinum-based chemotherapy with an ICI (preferably an PD-L1 blocking antibody) added. Platinum-based (preferably PE) chemotherapy will continue to destroy patients' tumour cells, but also potentially eliminate activated B cells and exhausted T cells. "Maintenance immunomodulation" will continue blocking immune checkpoints until the "second predictive biomarker" is matched, i.e., progression-free survival (PFS) lasts longer than its median (mPFS) for the respective tumour entity, or parameters of adaptive immune activation sink below the median threshold. PFS above median indicates that the ongoing anti-tumour immune response is losing its efficacy due to further immune editing.

Patients with liquid biopsy sample measurements matching the second predictive biomarker will then alternate "maintenance immunomodulation" from adaptive immunity by ICI maintenance to innate immunity by TLR9 agonist maintenance. Activation of antigen-presenting cells (APC), i.e., plasmacytoid dendritic cells (pDC) will be activated by TLR9 agonists, relay activation to myeloid dendritic cells and thereby restart tumour (neo-)antigen presentation for a new, not yet edited anti-tumour immune response.

Patients with liquid biopsy sample measurements classified as activated immune cells^{low}, (or below median) following induction or re-induction chemotherapy, likely reflect immunologically "cold" tumour micro-environments. In these tumours, "induction immunomodulation" and "maintenance immunomodulation" therapy by TLR9 agonists will improve OS by immune surveillance reactivation (ISR). ISR requires conversion of the immunologically "cold" TME into an immunologically "hot" environment. This is achieved during "maintenance immunomodulation" by TLR9 agonists. TLR9 activation will broadly activate innate immunity with its antigen presentation machinery (pDC and mDC) and effector cells (NK cells and NKT cells), but also T and B cell mediated adaptive immunity. If subsequently the **"second predictive biomarker"** is matched, i.e., progression-free survival (PFS) lasts longer than its median (mPFS) for the respective tumour entity, or adaptive immune activation parameters measured in a liquid biopsy rise above the median threshold. PFS above median indicates that the ongoing anti-tumour immune response is losing its efficacy due to its downregulation regulation by regulatory immune cells (Treg and Breg). Patients matching the second predictive biomarker will then alternate **"maintenance immunomodulation"** from innate immunity by TLR9 agonists to adaptive immunity by ICI. Blocking immune checkpoints by ICI will downregulate Treg and Breg activity and thereby allow for ongoing anti-tumour adaptive immunity.

| | |
|---|---|
| Table 1 | shows a summary of median overall survival (mOS), using mOS1 from IMPULSE (see Fig. 1) and an intention to treat (ITT) analysis. The respective treatment and control arms of IMPULSE, IMpower133, CASPIAN, Keynote-604, and EA5161 are shown. |
| Table 2. | shows regulatory T cells (%) descriptive statistics and pairwise comparisons; 2 sample t test, comparison of treatment groups; paired t test, differences to baseline within treatment groups. The fraction of regulatory T cells (Treg) increases with duration of TLR9 agonist treatment. PE chemo, platinum/etoposide for induction chemotherapy, followed by continuation maintenance until progress. Baseline, end of 4^{th} cycle of induction PE chemotherapy. Visit 2, days 118-125, Visit 3, days 146-153, Visit 4, days 188-195 calculated from start of induction PE chemotherapy, IMPULSE. |

**Table 1. Clinical study summary**

| **Clinical trial** | **IMPULSE** | **IMpower133** | **CASPIAN** | **Keynote-604** | **EA5161** |
|---|---|---|---|---|---|
| mOS treatment [m] | 12.4* | 12.3 | 13.0 | 10.8 | 11.3 |
| ITT [n] | 62 | 201 | 268 | 228 | 80 |
| mOS control [m] | 12.5* | 10.3 | 10.3 | 9.7 | 8.5 |
| ITT [n] | 41 | 202 | 269 | 225 | 80 |

**Table 2. Regulatory T cells (% of CD4+ T cells), IMPULSE**

| **Treatment** | **Visit** | **N** | **Mean** | **STD** | **Min** | **Median** | **Max** | **p value comp. to baseline** |
|---|---|---|---|---|---|---|---|---|
| PE chemo | Baseline | 34 | 8.0094 | 2.3313 | 4.04 | 7.760 | 14.63 | |
| **TLR9 agonist** | **Baseline** | 57 | 7.3905 | 2.0523 | 4.19 | **7.470** | 12.22 | |
| PE chemo | Visit 2 | 33 | 8.0766 | 1.9414 | 4.56 | 8.040 | 12.77 | 0.5986 |
| **TLR9 agonist** | **Visit 2** | 54 | 8.2827 | 2.0770 | 4.44 | **8.100** | 13.65 | **0.0002** |
| PE chemo | Visit 3 | 21 | 8.7771 | 2.0502 | 5.68 | 8.490 | 14.18 | 0.0538 |
| **TLR9 agonist** | **Visit 3** | 42 | 8.7502 | 1.9982 | 4.27 | **8.905** | 13.06 | **<0.0001** |
| PE chemo | Visit 4 | 11 | 8.9672 | 2.9203 | 5.22 | 7.960 | 16.39 | 0.2288 |
| **TLR9 agonist** | **Visit 4** | 20 | 9.5175 | 2.5398 | 5.82 | **9.205** | 16.33 | **0.0040** |

## Claims

1. A TLR9 agonist for use in treating cancer,
- wherein the TLR9 agonist is administered to a patient having undergone induction chemotherapy consisting of a first set of treatment cycles;
- wherein the patient after having undergone induction chemotherapy has been assigned a status of "non-matched-in-first" in a first predictive biomarker assay,
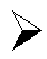 wherein said first predictive biomarker assay determines a percent of activated cells within an immune cell subset selected from B cells, T cells, natural killer T (NKT) cells, dendritic cells (DC), plasmacytoid dendritic cells (pDC), myeloid dendritic cells (mDC), and/or the percent of T regulatory (Treg) cells among CD4+ T cells in a blood sample obtained from the patient;
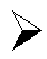 and wherein the patient is assigned a status of "non-matched-in-first" if the percent of activated cells within the immune subset is below (<) a median percent of activated cells, or < a median percent of Treg cells among CD4+ T cells,
- wherein subsequent to the induction chemotherapy, the TLR9 agonist is administered as part of a TLR9 agonist induction immunomodulation cycle,
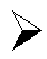 wherein a chemotherapy agent is administered in a first week of said TLR9 agonist induction immunomodulation cycle; and
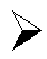 the TLR9 agonist is administered once per week, or twice per week in a second, and a third week of said TLR9 agonist induction immunomodulation cycle,
- and wherein, subsequent to a set of 1 to 4 of said TLR9 agonist induction immunomodulation cycles, the TLR9 agonist is administered as part of a TLR9 agonist maintenance immunomodulation regimen,
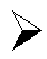 wherein the TLR9 agonist is administered twice per week, once per each week, or every two weeks for n weeks of said TLR9 agonist maintenance immunomodulation regimen, wherein n is an integer from 5 to 35, particularly from 9 to 18, more particularly n is 12.

2. The TLR9 agonist for use according to claim 1,
- wherein the patient has undergone the TLR9 agonist maintenance immunomodulation regimen as specified in claim 1 in the previous 3 weeks,
- wherein a second predictive biomarker assay determines a percent of Treg cells among CD4+ T cells in a blood sample obtained from the patient,
- wherein the patient is assigned a status of "non-matched-in-second" if the percent of activated Treg cells in the sample is < 20%,
and wherein subsequent to being assigned a status of "non-matched-in-second", the TLR9 agonist continues to be administered twice per week, once per week, or every 2 weeks for n weeks, wherein n is an integer from 6 to 24, particularly from 6 to 15, more particularly n is 9.

3. The TLR9 agonist for use according to claim 2,
- wherein in said second predictive biomarker assay as specified in claim 2:
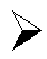 the patient is assigned a status of "non-matched-in-second" if the percent of Treg cells among CD4+ T cells in the sample is < 20%, or
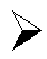 the patient is assigned a status of "matched-in-second" if the percent of Treg cells among CD4+ T cells in the sample is ≥ 20%,
- wherein subsequent to being assigned a status of "non-matched-in-second", the TLR9 agonist continues to be administered twice per week, once per week, or every 2 weeks for n weeks, wherein n is an integer from 6 to 24, particularly from 6 to 15, more particularly n is 9,
- or wherein subsequent to being assigned the status of "matched-in-second", and/or once the patient exceeds a median progression free survival threshold period from the beginning of the induction chemotherapy with no diagnosis of disease progression, particularly a median progression free survival threshold of about 188 days, a checkpoint modulator (CPM) is administered once every 2, or 3 weeks for n weeks, wherein n is an integer from 3 to 15.

4. The TLR9 agonist for use according to claim 2 or 3,
- wherein said second predictive biomarker assay is performed every 6 to 15 weeks, particularly every 9 weeks.

5. A CPM agent for use in treating cancer,
- wherein the CPM agent is administered to a patient having undergone induction chemotherapy consisting of a first set of treatment cycles;
- wherein the patient after having undergone induction chemotherapy has been assigned a status of "matched-in-first" in a first predictive biomarker assay which determines a percent of activated cells within an immune cell subset as specified in claim 1,
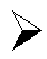 wherein the patient is assigned a status of "matched-in-first" if the percent of activated cells within the immune subset is ≥ a median percent of activated cells, or if the percent of Treg cells among CD4+ T cells ≥ a median percent of Treg cells among CD4+ T cells,
- wherein subsequent to the induction chemotherapy, the CPM agent is administered as part of a CPM induction immunomodulation cycle,
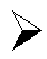 wherein a chemotherapy agent and a CPM agent are administered once in
the first week of said CPM induction immunomodulation cycle,
- and wherein, subsequent to a set of 1 to 5 of said CPM induction immunomodulation cycles, the CPM agent is administered as part of a CPM maintenance immunomodulation regimen,
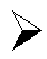 wherein the CPM agent is administered once every 2, or 3 weeks of said
CPM maintenance immunomodulation regimen for n weeks, wherein n is an integer from 6 to 36, particularly from 9 to 18, more particularly n is 12.

6. The CPM agent for use according to claim 5,
- wherein the patient has undergone of the CPM maintenance immunomodulation regimen as specified in claim 7 for the previous 3 weeks,
- wherein a second predictive biomarker assay determines the percent of Treg cells among CD4+ T cells in a blood sample obtained from the patient,
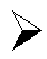 wherein the patient is assigned a status of "matched-in-second" if the percent of Treg cells among CD4+ T cells is ≥ 20%,
- and wherein subsequent to being assigned a status of "matched-in-second", the CPM agent is administered every 2, or 3 weeks for n weeks, wherein n is an integer from 6 to 36, particularly from 9 to 12, more particularly n is 12.

7. The CPM agent for use according to claim 6,
- wherein in said second predictive biomarker assay determined as specified in claim 6,
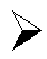 the patient is assigned a status of "non-matched-in-second" if the percent of Treg cells among CD4+ T cells in the sample is < 20%; or
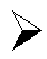 the patient is assigned a status of "matched-in-second" if the percent of activated Treg cells in the sample is ≥ 20%,
- wherein subsequent to being assigned a status of "non-matched-in-second", and/or once the patient exceeds a median progression free survival threshold period from the beginning of the induction chemotherapy with no diagnosis of disease progression, particularly a median progression free survival threshold of about 188 days, a TLR9 agonist is administered twice per week, once per week, or every 2 weeks for n weeks, wherein n is an integer from 6 to 24, particularly from 6 to 15, more particularly n is 9,
- or wherein subsequent to being assigned the status of "matched-in-second", the CPM agent is administered once every 2, or 3 weeks for n weeks, wherein n is an integer between 6 to 36, particularly from 9 to 12, more particularly n is 12.

8. The TLR9 agonist for use according to any one of the claims 2 to 4, or the CPM agent for use according to any one of claims 6 or 7,
- wherein said second predictive biomarker assay is performed every 6 to 15 weeks, particularly every 9 weeks,
- wherein if the patient has undergone the CPM maintenance immunomodulation regimen as specified in claim 5 in the previous 3 weeks, then 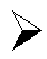 subsequent to being assigned a status of "non-matched-in-second", and/or once the patient exceeds a median progression free survival threshold period from the beginning of the induction chemotherapy with no diagnosis of disease progression, particularly a median progression free survival threshold of about 188 days, the TLR9 agonist is administered twice per week, once per week, or every 2 weeks for the subsequent n weeks, wherein n is an integer from 6 to 24, particularly from 6 to 15, more particularly n is 9; or
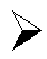 subsequent to being assigned the status of "matched-in-second", the CPM continues to be administered once every 2, or 3 weeks for the subsequent n weeks, wherein n is an integer from 6 to 24, particularly from 6 to 12, more particularly n is 9;
or
- wherein if the patient has undergone the TLR9 maintenance immunomodulation regimen as specified in claim 1 for the previous 3 weeks;
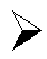 subsequent to being assigned a status of "non-matched-in-second", the TLR9 agonist continues to be administered twice per week, once per week, or every 2 weeks for the subsequent n weeks, wherein n is an integer from 6 to 24, particularly from 6 to 15, more particularly n is 9; or
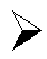 subsequent to being assigned the status of "matched-in-second", and/or once the patient exceeds a median progression free survival threshold period from the beginning of the induction chemotherapy with no diagnosis of disease progression, particularly a median progression free survival threshold of about 188 days, the CPM is administered once every 2, or 3 weeks for the subsequent n weeks, wherein n is an integer from 6 to 24, particularly from 6 to 12, more particularly n is 9.

9. The TLR9 agonist for use according to any one of claims 1 to 4, or 8, or the CPM agent for use according to any one of claims 7 or 8, wherein the TLR9 agonist is an oligodeoxynucleotide comprising an unmethylated CG dinucleotide,
particularly wherein
- the unmethylated CG dinucleotide is N¹N²CGN³N⁴, wherein N¹N² is AA, TT, GG, GT, or AT, and N³N⁴ is CT, TT, TC, TG, or CG, and/or
- the TLR-9 agonist comprises a stretch of at least three or four consecutive deoxyguanosines, and/or
- the TLR9 agonist is a dumbbell deoxynucleotide,
even more particularly wherein the TLR9 agonist is Leftitolimod (CAS: 1548439-51-5).

10. The TLR9 agonist for use according to any one of claims 1 to 4, 8, or 9, or the CPM agent for use according to any one of claims 7 to 9, wherein the TLR9 agonist is administered by means of subcutaneous injection:
- twice per week at a dose of 50-80 mg, particularly 60 mg with a 2, or 3 day interval,
- once per week at a dose of 30-60 mg, particularly 60 mg,
- once per week at a dose of 100 to 150 mg, particularly 120 mg, or
- once every 2 weeks at a dose of 100 to 150 mg, particularly 120 mg,
particularly wherein the TLR9 agonist is administered once per week at a dose of 60 mg.

11. The TLR9 agonist for use according to any one of claims 1, to 4, or 8 to 10, or the CPM agent for use according to any one of claims 5 to 10, wherein the checkpoint modulator is is an antibody specific for PD-1 or PDL-1, particularly an antibody selected from atezolizumab, durvalumab, pembrolizumab, or nivolumab, more particularly an anti-PD-L1 antibody selected from atezolizumab or durvalumab.

12. The TLR9 agonist, or the CPM agent for use according to any one of the preceding claims, wherein the cancer is a cancer sensitive to chemotherapy agent in combination with an immune adjuvant treatment, particularly a cancer selected from breast cancer, cervical cancer, endometrial cancer, colorectal cancer, metastatic colorectal carcinoma, or lung cancer, more particularly small cell lung cancer or metastatic colorectal cancer, still more particularly extensive stage small cell lung cancer (SCLC).

13. The TLR9 agonist, or the CPM agent for use according to any one of the preceding claims, particularly for use in treatment of lung cancer, more particularly in treatment of extensive stage SCLC, wherein the patient is assigned
- a status of "non-matched-in-first" is assigned to the patient when the result of the first predictive biomarker is compared to a threshold selected from:
i. a ratio of activated B cells < 16.9% of B cells,
ii. a ratio of activated T cells < 0.76% of T cells,
iii. a ratio of Treg cells < 7.6% of CD4+ T cells,
iv. a ratio of activated NKT cells < 4.1% of NK cells,
v. a ratio of activated mDC is < 0.48% of pDC
vi. a ratio of activated pDC is < 5.0% of pDC, and/or
vii. a ratio of activated DC < 54.51% of DC, or
- a status of "matched-in-first" is assigned to the patient when the result of the first predictive biomarker is compared to a threshold selected from:
i. a ratio of activated B cells ≥ 16.9% of B cells, particularly ≥ 21% of B cells,
ii. a ratio of activated T cells ≥ 0.76% of T cells,
iii. a ratio of activated Treg cells ≥ 7.6% of Treg cells, and/or
iv. a ratio of activated NKT cells ≥ 4.1% of NK cells,
v. a ratio of activated mDC is ≥ 0.48% of pDC
vi. a ratio of activated pDC is ≥ 5.0% of pDC, and/or
vii. a ratio of activated DC ≥ 54.51% of DC;
particularly wherein the percent of activated B cells is determined in the first predictive biomarker assay.

14. The TLR9 agonist, or the CPM agent for use according to any one of the preceding claims, wherein the induction chemotherapy consists of a first set of treatment cycles comprising
- 2, 3, 4, or 5 cycles, particularly 4 cycles, wherein in each cycle consists of three weeks,
- and wherein a chemotherapeutic agent, or combination of chemotherapeutic agents is administered in the first week of said induction chemotherapy cycle,
- particularly wherein the induction chemotherapy cycle consists of administration of a platinum-based combination of chemotherapy agents in the first week of said induction chemotherapy cycle.

15. The TLR9 agonist, or the CPM agent for use according to any one of the preceding claims, wherein the induction chemotherapy comprises or consists of treatment with
- an antineoplastic platinum complex drug, particularly an antineoplastic platinum complex drug selected from cisplatin, oxaliplatin, or carboplatin, and
- an antineoplastic alkaloid drug, particularly an antineoplastic alkaloid drug selected from etoposide or topotecan.
